(19) Europäisches Patentamt
European Patent Office
Office européen des brevets

(11) EP 3 644 320 A1

(12) EUROPEAN PATENT APPLICATION

(43) Date of publication:
29.04.2020 Bulletin 2020/18

(51) Int Cl.:
G16H 20/10 (2018.01)   A24F 40/00 (2020.01)

(21) Application number: 19205350.2

(22) Date of filing: 25.10.2019

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
KH MA MD TN

(30) Priority: 26.10.2018   US 201862751292 P

(71) Applicant: Canopy Growth Corporation
Smiths Falls, Ontario K7A 0A8 (CA)

(72) Inventors:
• DAVIS, STEPHEN
OTTAWA, K2J 1G8 (CA)

• GUAY, SHANE
KANATA, K2K 3M7 (CA)
• PENNEY, STEVEN
OTTAWA, K2M 2J7 (CA)
• POPPLEWELL, PETER
OTTAWA, K2J 1T6 (CA)
• STEWART, ANDREW
OTTAWA, K2J 1G8 (CA)

(74) Representative: Murgitroyd & Company
Murgitroyd House
165-169 Scotland Street
Glasgow G5 8PL (GB)

(54) **VAPORIZER SYSTEM WITH DOSE-METERING FOR REDUCING CONSUMPTION OF A SUBSTANCE**

(57) The present invention is directed to a system for tracking and reducing consumption of a substance by a user. The system comprises a vape device comprising a payload reservoir configured to contain a payload to be vaporized, wherein the payload includes the substance, and an atomizer configured to vaporize a portion of the payload during each of a plurality of user inhalations. The system also comprises a software application executable on the computing device, wherein the software application causes the computing device to determine a dosage schedule that causes a reduction in consumption of the substance over a period of time, and control operation of the vape device in accordance with the dosage schedule during a substance reduction period.

FIG. 1

## Description

### CROSS-REFERENCE TO RELATED APPLICATIONS

This application is based on and claims priority to U.S. Provisional Application Serial No. 62/751,292, filed on October 26, 2019, which is incorporated herein by reference in its entirety.

### STATEMENT REGARDING FEDERALLY SPONSORED RESEARCH OR DEVELOPMENT

Not applicable.

### BACKGROUND OF THE INVENTION

### 1. FIELD OF THE INVENTION

The present disclosure is generally related to the field of personal vaporizer devices and, in particular, to a system for tracking and controlling the dosage of a vaporized payload that is delivered to the user of a personal vaporizer device so as to reduce consumption of a substance in the payload.

### 2. DESCRIPTION OF RELATED ART

The use of personal vaporizer devices or "vape devices" for consuming tobacco products, cannabis, and other substances has grown significantly. In a basic form, a vape device consists of an atomizer, a battery, a switch for connecting the battery to the atomizer, and a reservoir that contains an amount of payload to be vaporized by the atomizer. Controlling the vape device merely entails closing the switch so that current passes from the battery through a coil of the atomizer whereby the atomizer heats up and begins to vaporize a portion of the payload. The vapor-i.e., the cloud-like emission from a vape device that may be some combination of actual gas phase vapor and aerosol-is then inhaled by the user so that the desired substances (e.g., nicotine, tetrahydrocannabinol (THC), cannabidiol (CBD), etc.) are delivered for medical or recreational purposes.

Vape devices for vaporizing a payload that includes nicotine are being widely marketed as smoking cessation devices. However, in many cases, these vape devices simply cause a user to convert from inhaling nicotine due to the burning of tobacco (i.e., smoking) to inhaling an equivalent and sometimes increased dosage of vaporized nicotine. Also, most of these vape devices offer poor dose metering performance and, typically, a user must rely on the volume of liquid payload used over a period of time to have a general sense of the amount of nicotine being consumed. Thus, there remains a need in the art for a vape device that accurately measures and controls the dose of a vaporized payload delivered to a user so as to reduce consumption of nicotine or other substances in the payload.

### BRIEF SUMMARY OF THE INVENTION

The present invention is directed to a system comprising a vape device in communication with a computing device for tracking and reducing consumption of a substance by a user. In general terms, the vape device is configured to enable consumption of a payload that includes the substance during a plurality of user inhalations (each of which is commonly referred to as a "draw" or "drag" or "puff"), and the computing device is configured to control operation of the vape device so as to cause a reduction in consumption of the substance over a period of time.

A system for tracking and reducing consumption of a substance by a user in accordance with one exemplary embodiment of the invention described herein comprises a vape device and a software application executable on a computing device. The vape device is configured to enable consumption of the substance during a plurality of user inhalations, wherein the vape device comprises: (a) a payload reservoir configured to contain a payload to be vaporized, wherein the payload includes the substance; and (b) an atomizer configured to vaporize a portion of the payload during each of the user inhalations. The software application causes the computing device to: (a) determine a dosage schedule that causes a reduction in consumption of the substance over a period of time; and (b) control operation of the vape device in accordance with the dosage schedule during a substance reduction period.

A computing device for tracking and reducing consumption of a substance by a user in accordance with another exemplary embodiment of the invention described herein comprises a processor, a memory device, and a set of instructions stored in the memory device and executable by the processor to: determine a dosage schedule that causes a reduction in consumption of the substance over a period of time; and control operation of an atomizer in accordance with the dosage schedule during a substance reduction period.

A system for tracking and reducing consumption of a substance by a user in accordance with another exemplary embodiment of the invention described herein comprises a vape device and a software application executable on a computing device. The vape device is configured to enable consumption of a first substance and a second substance during a plurality of user inhalations, wherein the vape device comprises: (a) a first payload reservoir configured to contain a first payload to be vaporized, wherein the first payload includes the first substance; (b) a first atomizer configured to vaporize a portion of the first payload during a plurality of the user inhalations; (c) a second payload reservoir configured to contain a second payload to be vaporized, wherein the second payload includes the second substance; and (d) a second atomizer configured to vaporize a portion of the second payload during a plurality of the user inhalations. The software application causes the computing device to: (a) determine a dosage schedule that causes a reduction in consumption of the first substance over a period of time and, optionally, to cause an increase in consumption of the second substance over the period of time; and (b) control operation of the vape device in accordance with the dosage schedule during a substance reduction period.

A computing device for tracking and reducing consumption of a first substance and a second substance by a user in accordance with yet another exemplary embodiment of the invention described herein comprises a processor, a memory device, and a set of instructions stored in the memory device and executable by the processor to: determine a dosage schedule that causes a reduction in consumption of the first substance over a period of time and, optionally, to cause an increase in consumption of the second substance over the period of time; and control operation of first and second atomizers in accordance with the dosage schedule during a substance reduction period.

**BRIEF DESCRIPTION OF THE DRAWINGS**

FIG. 1 is a schematic diagram of a vape device in accordance with a first exemplary embodiment of the invention described herein.

FIG. 2 is a schematic diagram of a vape device in accordance with a second exemplary embodiment of the invention described herein.

FIG. 3 is a schematic diagram of a vape device system that includes a vape device in wireless communication with a computing device.

FIG. 4 is a schematic diagram of a vape device that utilizes a capacitive vapor measurement method to determine the dose of vaporized payload delivered to a user in accordance with one embodiment of the invention described herein.

FIG. 5 is a front elevational view of a rolled capacitor that may be used as a capacitive sensor in the vape device of FIG. 4.

FIG. 6 is a front elevational view of the rolled capacitor of FIG. 5 with arrows indicating the direction of airflow in a plane parallel to the front face of the rolled capacitor.

FIG. 7 is a perspective view of the rolled capacitor of FIG. 5 with arrows indicating the direction of airflow in a plane perpendicular to the front face of the rolled capacitor.

FIG. 8 is a front elevational view of an interdigitated capacitor that may be used as a capacitive sensor in the vape device of FIG. 4.

FIG. 9 is a front elevational view of the interdigitated capacitor of FIG. 8 with arrows indicating the direction of airflow in a plane parallel to the front face of the interdigitated capacitor.

FIG. 10 is a front elevational view of the interdigitated capacitor of FIG. 8 with arrows indicating the direction of airflow in a plane perpendicular to the front face of the interdigitated capacitor.

FIG. 11 is a schematic diagram of a vape device that utilizes a first light intensity measurement method to determine the dose of vaporized payload delivered to a user in accordance with one embodiment of the invention described herein.

FIG. 12 is a schematic diagram of a vape device that utilizes a second light intensity measurement method to determine the dose of vaporized payload delivered to a user in accordance with one embodiment of the invention described herein.

FIG. 13 is a schematic diagram of a vape device that utilizes a third light intensity measurement method to determine the dose of vaporized payload delivered to a user in accordance with one embodiment of the invention described herein.

FIG. 14 is a schematic diagram of a vape device that utilizes a fourth light intensity measurement method to determine the dose of vaporized payload delivered to a user in accordance with one embodiment of the invention described herein.

FIG. 15 is a schematic diagram of a vape device that utilizes a fifth light intensity measurement method to determine the dose of vaporized payload delivered to a user in accordance with one embodiment of the invention described herein.

FIGS. 16A-16C are schematic diagrams of a vape device that utilizes a sixth light intensity measurement method to determine the dose of vaporized payload delivered to a user in accordance with one embodiment of the invention described herein.

FIG. 17 is a schematic diagram of a vape device that utilizes a seventh light intensity measurement method to determine the dose of vaporized payload delivered to a user in accordance with one embodiment of the invention described herein.

FIG. 18 is a schematic diagram of a vape device that utilizes an eighth light intensity measurement method to determine the dose of vaporized payload delivered to a user in accordance with one embodiment of the invention described herein.

FIG. 19 is a block diagram of a vape device operable to enable consumption of a single substance in accordance with one embodiment of the invention described herein, wherein the dose of the substance decreases over a period of time.

FIG. 20 is a screen shot of a computing device with a graphical user interface that shows the average daily usage of a substance during a calibration period in accordance with one embodiment of the invention described herein.

FIG. 21 is a screen shot of a computing device with a graphical user interface that enables a user to set a goal for reducing consumption of a substance in accordance with one embodiment of the invention described herein.

FIG. 22 is a screen shot of a computing device with a graphical user interface that enables a user to track progress toward a goal for reducing consumption of a substance in accordance with one embodiment of the invention described herein.

FIG. 23 is a block diagram of a vape device operable to enable consumption of first and second substances in accordance with one embodiment of the invention described herein, wherein the dose of the first substance decreases over a period of time and, optionally, the dose of the second substance increases over the period of time.

## DETAILED DESCRIPTION OF EXEMPLARY EMBODIMENTS

The present invention is directed to a system comprising a vape device in communication with a computing device for tracking, modifying and metering the dosage of vaporized payload delivered to a user so as to reduce the user's consumption of a substance over a period of time. In some embodiments, the system is used to dispense nicotine in precisely metered doses that allow the user to control his or her cravings and reduce consumption gradually and safely. In other embodiments, the system is used to reduce a user's consumption of THC to a desired dosage. In yet other embodiments, the system is used to vaporize at least two substances, i.e., a first substance whose consumption is desired to be reduced (e.g., nicotine or THC) and a second substance (e.g., CBD), wherein the consumption of the second substance is optionally increased over the period of time to replace all or a portion of the volume of the first substance.

While the invention will be described in detail below with reference to various embodiments, it should be understood that the invention is not limited to the specific configuration or methodologies of any of these embodiments. In this description, references to "one embodiment," "an embodiment," "an exemplary embodiment," or "embodiments" mean that the feature or features being described are included in at least one embodiment of the invention. Separate references to "one embodiment," "an embodiment," "an exemplary embodiment," or "embodiments" in this description do not necessarily refer to the same embodiment and are also not mutually exclusive unless so stated and/or except as will be readily apparent to those skilled in the art from the description. For example, a feature, structure, function, etc. described in one embodiment may also be included in other embodiments, but is not necessarily included. Thus, the present invention can include a variety of combinations and/or integrations of the embodiments described herein.

### I. Vape Device

The system of the present invention includes a vape device configured to measure and meter the dose of vaporized payload inhaled by the user through communication with a computing device (described below). The invention may be implemented with a variety of different types of vape devices available in various sizes in terms of the amount of payload they can contain. In one embodiment, the vape device comprises a self-contained vape device, e.g., a one piece disposable vape device in which all of the components are contained within a single housing. In another embodiment, the vape device comprises a control assembly and cartridge that are formed in separate housings and releasably connected to each other via an electromechanical connection. In this embodiment, the control assembly is provided as a re-useable component that can be used with multiple disposable cartridges. In yet another embodiment, the vape device comprises a tabletop or desktop vaporizer.

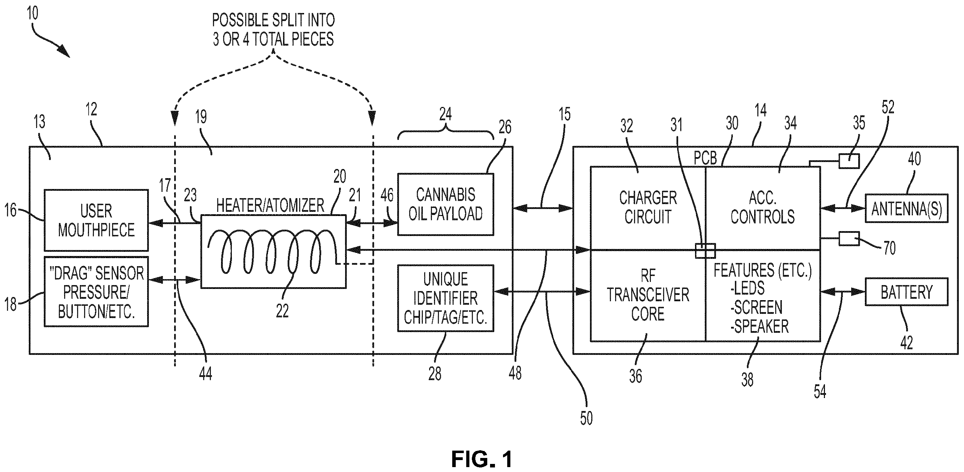

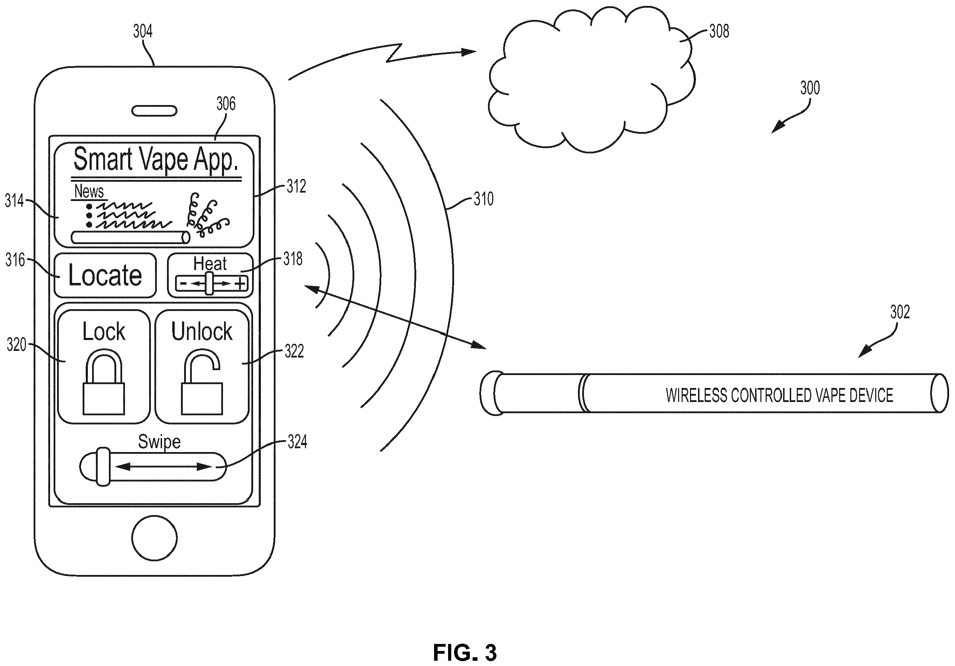

Various embodiments of vape devices that may be utilized to implement the present invention are described below in connection with FIGS. 1-2, 4-19 and 23. The vape devices communicate with a computing device and work interactively with a software application or "app" operating on the computing device to provide various functions and features that enable implementation of certain aspects of the invention, as described below in connection with FIGS. 3 and 20-22. Of course, it should be understood that the present invention is not limited to these embodiments and that other types of vape devices may also be used within the scope of the invention.

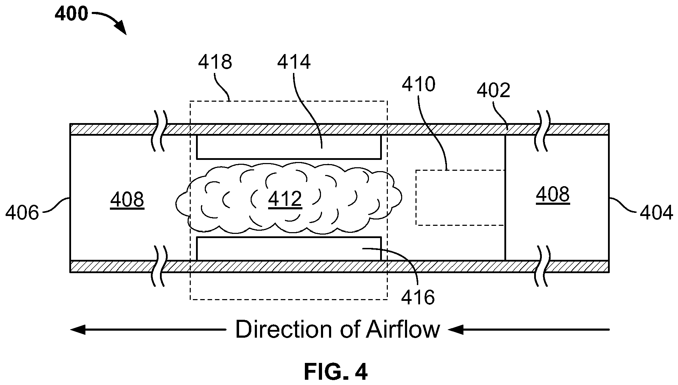

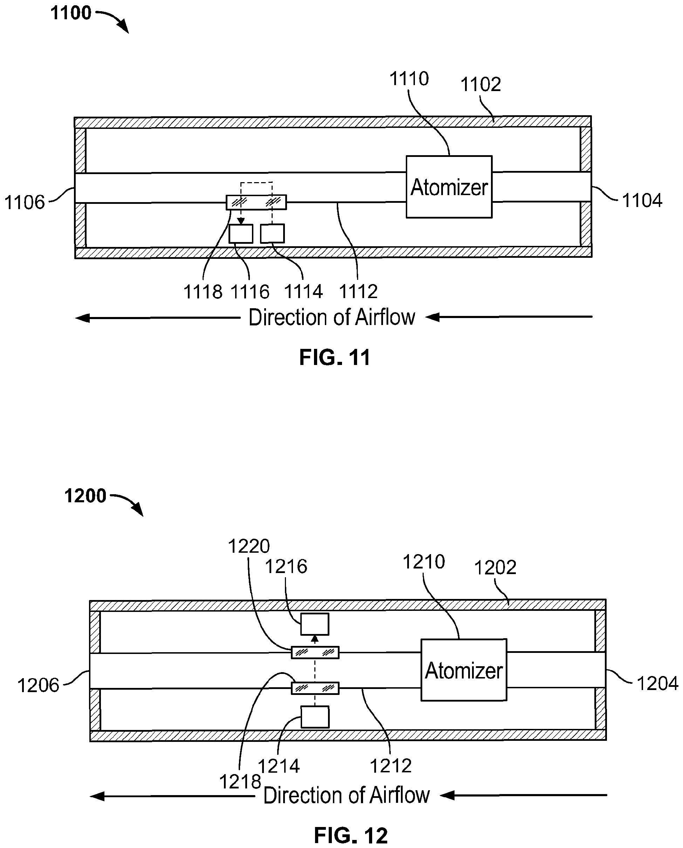

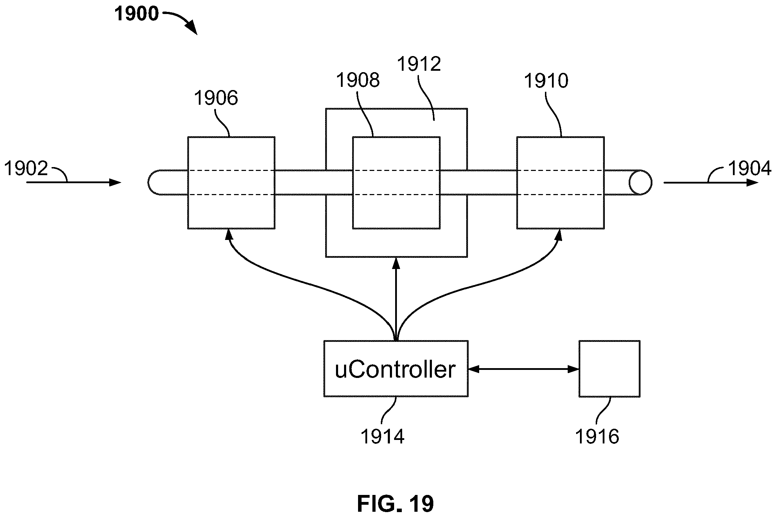

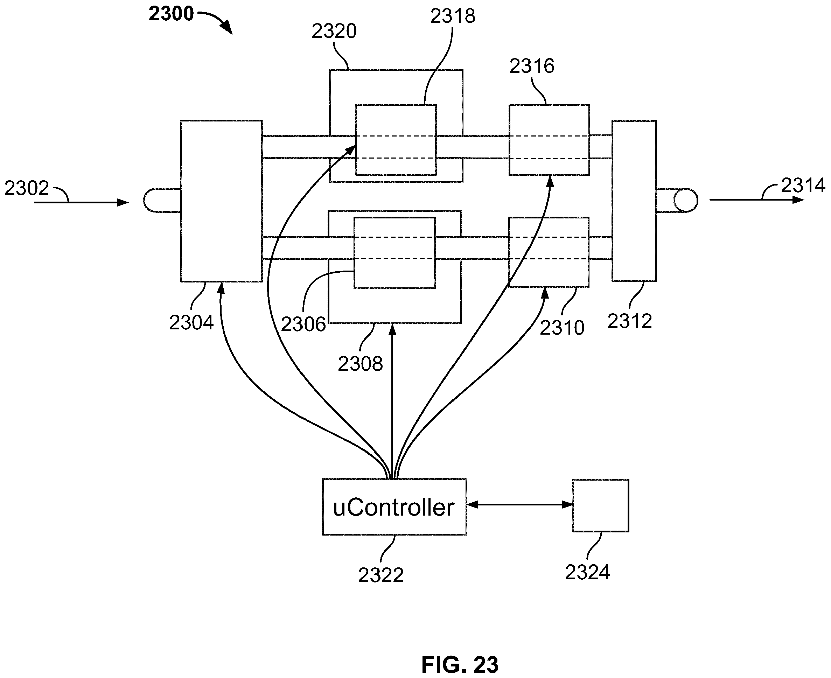

For the sake of simplicity, the vape devices shown in FIGS. 1-2 are provided to describe the general structural configuration of the vape devices and do not include all of the various components and circuits required to provide the dose measurement technology required to implement the present invention; rather, these components and circuits are described below in connection with the vape devices shown in FIGS. 4-10 (which include various capacitive vapor measurement systems) and FIGS. 11-18 (which include various light intensity measurement systems). Also, operation of the vape device is described below in connection with the vape devices shown in FIG. 19 (which may be used to vaporize a single payload) and FIG. 23 (which may be used to vaporize two different payloads).

### First Embodiment of Vape Device

Referring to FIG. 1, a first embodiment of a vape device is shown generally as reference numeral 10. Vape device 10 includes a mouthpiece assembly 12, an atomizer assembly 19, a payload assembly 24, and a control assembly 14. Any of mouthpiece assembly 12, atomizer assembly 19, payload assembly 24, and control assembly 14 may be formed integrally together and included within a common housing suitable for grasping by a user. Further, any of mouthpiece assembly 12, atomizer assembly 19, payload assembly 24, and control assembly 14 may be formed in separate housings that are releasably connected to each other via connecting means 15, which can comprise, for example, one or more of pressure or friction fit connection means, twist mechanical lock means, magnetic connection means and any other connecting means as well known to those skilled in the art. The connecting means 15 may include a female 510 threaded connector on the control assembly 14 that releasably engages a male 510 threaded connector on the atomizer assembly 19 or payload assembly 24. A 510 threaded connector, as is known in the art, is a M7-0.5x5 threaded connector, i.e., a threaded connector with a nominal diameter of 7 mm, a pitch of 0.5 mm, and a length of 5 mm. Connecting means 15 may include threaded connectors of other sizes. By way of example, mouthpiece assembly 12 may be releasably connected to atomizer assembly 19, payload assembly 24 and control assembly 14, which are either formed integrally together or in separate housings that are releasably connected to each other. Mouthpiece assembly 12 and atomizer assembly 19 may be formed integrally together and releasably connected to payload assembly 24 and control assembly 14, which are either formed integrally together or in separate housings that are releasably connected to each other. Further, mouthpiece assembly 12, atomizer assembly 19, and payload assembly 24 may be formed integrally together and releasably connected to control assembly 14. The combination of the mouthpiece assembly 12, atomizer assembly 19, and payload assembly 24 may be referred to as a cartridge herein. It is also within the scope of the invention for the mouthpiece assembly 12 to be omitted and for the vaporized payload to exit the atomizer assembly 19 directly for inhalation.

In some embodiments, a heater or atomizer 20 is disposed in atomizer assembly 19, with atomizer 20 further comprising a heating element 22 disposed therein for heating and vaporizing a payload that may comprise, for example, liquids, oils or other fluids (e.g., nicotine oil or cannabis oil). Vape device 10 may also be modified to vaporize a tablet of dry material or dry material that is not in tablet form (e.g., ground cannabis bud). Heating element 22 may be a heating coil. Atomizer 20 can comprise an inlet 21 and an outlet 23, wherein inlet 21 can be in communication, via fluid connector 46, with payload reservoir 26 disposed in payload assembly 24, wherein payload reservoir 26 can contain a payload for vaporization or atomization. Outlet 23 can be in communication with a user mouthpiece 16 of mouthpiece assembly 12 via a conduit 17, which is typically a hollow tube made of stainless steel, aluminum, or other materials known to those skilled in the art.

In some embodiments, payload assembly 24 contains a radio frequency identification (RFID) tag 28. RFID tag 28 may be any type of device that includes memory or storage capable of storing a payload identifier that identifies payload reservoir 26 and/or other information related to the payload contained in payload reservoir 26, as discussed below. RFID tag 28 also includes means for allowing the stored information to be retrieved by another device, such as an RFID reader in communication with microcontroller 31. Microcontroller 31 may process the information retrieved from RFID tag 28 and/or transmit the information to an external computing device via a radio frequency (RF) transceiver circuit 36 and antenna(s) 40. In one embodiment, RFID tag 28 comprises an integrated circuit (IC) chip for modulating and demodulating radio frequency signals, such as a galvanically isolated near field communication (NFC) tag that can be read by any NFC-capable device. In one embodiment, the NFC tag is read directly by an external computing device, such as computing device 72 described below. Of course, other short-range wireless technologies may also be used in accordance with the present invention.

In some embodiments, mouthpiece assembly 12 can comprise a draw sensor 18 operatively coupled to atomizer 20 via an electrical connection 44, wherein draw sensor 18 can cause a power signal (e.g., a direct current or pulsed direct current) to flow from battery 42 through heating element 22. In some embodiments, draw sensor 18 comprises a sensor, such as a mass air flow sensor, that can produce an electrical signal in response to when a user inhales or draws on mouthpiece 16, wherein the electrical signal can cause the power signal to flow from battery 42 through heating element 22. In some embodiments, draw sensor 18 can be used as a simple "switch" as a means to turn on atomizer 20 to vaporize payload drawn into atomizer 20 from payload reservoir 26 as the user draws on mouthpiece 16. Draw sensor 18 is one type of activation mechanism that may be used to activate atomizer 20. Draw sensor 18 may be replaced with or used in connection with another type of activation mechanism that receives an input to switch it from an "off position, in which atomizer 20 is not activated, and an "on" position, in which atomizer 20 is activated. For example, draw sensor 18 may be replaced with or used in connection with any of the following types of activation mechanisms: a button, switch, pressure transducer, proximity sensor, flow sensor, touch sensor, voice recognition sensor, haptic control, saliva and breath biosensor, and the like.

In some embodiments, mouthpiece 16 and draw sensor 18 can be part of a single-piece mouthpiece assembly 12, or can be disposed in a separate mouthpiece section 13 that forms part of mouthpiece assembly 12.

In some embodiments, atomizer 20 can be disposed in atomizer assembly 19 that can either be integral to mouthpiece assembly 12, or a physically separate enclosure that can couple to mouthpiece assembly 12. Instead of or in addition to including a heating element 22 as disclosed herein, atomizer 20 may include any other structure capable of vaporizing or atomizing a payload in a suitable form for inhalation. For example, atomizer 20 may include a jet nebulizer, an ultrasonic nebulizer, or a mesh nebulizer.

In some embodiments, payload reservoir 26 and RFID tag 28 can be disposed in payload assembly 24 that can either be integral to mouthpiece assembly 12 and/or atomizer assembly 19, or a physically separate enclosure that can couple to mouthpiece assembly 12 and/or atomizer assembly 19, which can include one or more of connecting means 15 described above. Preferably, RFID tag 28 is physically coupled to payload reservoir 26 either directly or indirectly (e.g., RFID tag 28 and payload reservoir 26 are included in a common housing of payload assembly 24) in a tamper resistant manner.

In some embodiments, control assembly 14 can comprise one or more antennas 40, a power source such as battery 42, and a printed circuit board 30 that can further comprise a microcontroller 31 configured for carrying out one or more electronic functions in respect of the operation of vape device 10. Having more than one antenna 40 can enable the ability for diversity wireless communications of RF signals, as well known to those skilled in the art. In some embodiments, battery 42 can comprise a lithium ion power cell battery, although other battery technologies can be used as well known to those skilled in the art. As the vape devices are personal use devices, the battery 42 can comprise technology that prevents the advent of an explosion should the battery fail.

In some embodiments, circuit board 30 can comprise a charger circuit 32 configured for charging battery 42. Charger circuit 32 can be integral to circuit board 30 or can be disposed on a separate circuit board operatively connected to circuit board 30 and to battery 42 via electrical connection 54. Charger circuit 32 can be configured to be operatively connected to an external source of power, either via a shared or dedicated electrical connector 35 operatively coupled to circuit board 30 with internal connection to charger circuit 32, or a wireless connection for power transfer, as well known to those skilled in the art.

In some embodiments, circuit board 30 can comprise user input interface circuit 34 and output interface circuit 38. Either or both of input interface circuit 34 and output interface circuit 38 can be integral to circuit board 30 or can be disposed on a separate circuit board operatively connected to circuit board 30. In some embodiments, input interface circuit 34 can provide the electrical interface between user controls and activation mechanisms disposed on vape device 10, such as buttons, switches, draw sensors, pressure transducers, proximity sensors, flow sensors, touch sensors, voice recognition sensors, haptic controls, saliva and breath biosensors, and the like, and microcontroller 31 and, thus, can provide the means to relay user input commands from the user controls as instructions to microcontroller 31 to operate vape device 10. For example, input interface circuit 34 may be electrically coupled to draw sensor 18 for receiving an "on" signal from draw sensor 18 when a user draws on mouthpiece 16. When input interface circuit 34 receives the "on" signal from draw sensor 18, it may send instructions to microcontroller 31 to cause atomizer 20 to provide vapor through outlet 23 by supplying a controlled current or voltage to heating element 22, provided that any other conditions necessary to activate atomizer 20 have been met.

In some embodiments, output interface circuit 38 can provide the electrical interface between microcontroller 31 and output display devices, such as indicator lights, alphanumeric display screens, audio speakers, surface heaters, vibration devices, and any other forms of tactile feedback devices as well known to those skilled in the art, and, thus, can provide the means to relay information relating to the operation of vape device 10 from microcontroller 31 to the user.

In some embodiments, circuit board 30 can comprise an RF transceiver circuit 36 to provide the means for wireless communication of data between vape device 10 and a computing device, such as computing device 72 as shown in FIG. 3. In some embodiments, RF transceiver circuit 36 can be integral to circuit board 30 or can be disposed on a separate circuit board operatively connected to circuit board 30. RF transceiver circuit 36 can be connected to one or more antennas 40 via electrical connection 52, as well known to those skilled in the art. RF transceiver circuit 36 and the one or more antennas 40 comprise a wireless transceiver of vape device 10.

In some embodiments, microcontroller 31 can comprise a microprocessor (which for purposes of this disclosure also incorporates any type of processor) having a central processing unit as well known to those skilled in the art, wherein the microprocessor can further comprise a memory configured for storing a series of instructions for operating the microprocessor in addition to storing data collected from sensors disposed on vape device 10 or data received by vape device 10 to control its operation, such as operational settings or dosage information. Microcontroller 31 is in electrical communication with charger circuit 32, user input interface circuit 34, output interface circuit 38, and RF transceiver circuit 36 for receiving instructions and/or data from and/or transmitting instructions and/or data to charger circuit 32, user input interface circuit 34, output interface circuit 38, and RF transceiver circuit 36.

In some embodiments, atomizer 20 can be operatively and electrically connected to circuit board 30 via electrical connection 48, which can provide the means to activate atomizer 20 (e.g., deliver electrical current from battery 42 to heating element 22) when an activation mechanism such as draw sensor 18 sends an "on" signal to microcontroller 31, as well as receiving data signals from draw sensor 18 and/or atomizer 20. In this manner, the activation mechanism (i.e., draw sensor 18) is coupled to the atomizer 20 indirectly through microcontroller 31, and a direct connection between the activation mechanism and atomizer 20 is not required (i.e., the activation mechanism sends a signal to microcontroller 31 which sends a signal to activate atomizer 20). In addition to controlling operation of atomizer 20 based on a signal received from the activation mechanism, microcontroller 31 also controls operation of atomizer 20 based on the operational settings or dosage information described below. In some embodiments, microcontroller 31 can be operatively connected to RFID tag 28 via electrical connection 50.

As used herein, the term "electrical connection" shall include any form of electrical connection via a wired or wireless connection, such as electrical conductors or wires suitable for the transmission of a power signal (e.g., a direct current or pulsed direct current), analog or digital electrical signals or radio frequency signals, as the case may be and as well-known to those skilled in the art.

The operational settings referred to herein include any type of setting or instruction that instructs the vape device 10 or certain components of the vape device 10 to operate or not operate in a particular manner. Specifically, operational settings of the vape device 10 include one or more of a duty cycle setting, a temperature setting, and an operational time duration. The duty cycle setting preferably corresponds to a pulse width modulation instruction transmitted from microcontroller 31 to battery 42 to send electrical current to heating element 22 in a particular desired manner. The temperature setting preferably corresponds to a temperature instruction transmitted from microcontroller 31 to battery 42 to send electrical current to heating element 22 to maintain heating element 22 at a desired temperature or range of temperatures. A temperature sensor may be coupled to microcontroller 31 to measure the actual temperature of heating element 22 and transmit that information to microcontroller 31 for determination of the amount and duration of electrical current that needs to be sent to heating element 22 to maintain a particular temperature or range of temperatures. The operational time duration preferably corresponds to a time instruction transmitted from microcontroller 31 to battery 42 to maintain heating element 22 at a temperature suitable for vaporization of the contents of payload reservoir 26 for a desired time.

The dosage information referred to herein preferably corresponds to a dosage instruction transmitted from microcontroller 31 to battery 42 that powers down heating element 22 when a desired volume of vapor passes through atomizer 20. As described in greater detail below, various dose measurement methods may be used to accurately measure the volume of vaporized payload passing through atomizer 20 to mouthpiece 16 for user inhalation, whereby microcontroller 31 compares the actual volume passed through atomizer 20 to the dosage setting to determine when to shut off heating element 22.

### Second Embodiment of Vape Device

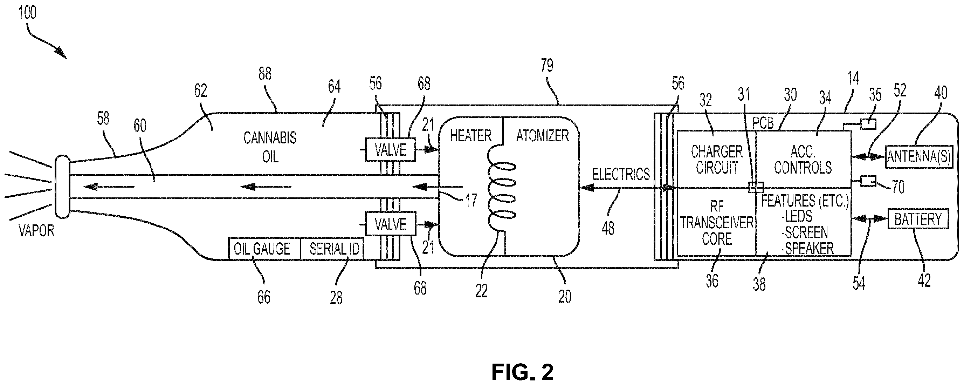

Referring to FIG. 2, a second embodiment of a vape device is shown generally as reference numeral 100. In some embodiments, vape device 100 can comprise control assembly 14, atomizer assembly 79 and mouthpiece assembly 88 operatively coupled together in that order using mechanical connection means 56 to join the subassemblies together. Mechanical connection means 56 can comprise one or more of threaded connection means, magnetic connection means and friction or press-fit connection means, and any of the connection means 15 described above, including 510 threaded connectors. In some embodiments, mouthpiece assembly 88 can comprise a mouthpiece 58 in communication with the outlet of atomizer 20 via conduit 60, which is typically a hollow tube made of stainless steel, aluminum, or other materials known to those skilled in the art. Mouthpiece assembly 88 can further comprise a payload reservoir 62 that can be filled with a payload 64 that may be liquid or oil. The payload 64 can flow from payload reservoir 62 to inlet 21 of atomizer 20 via one or more valves 68. In some embodiments, mouthpiece assembly 88 can comprise RFID tag 28 and an oil gauge 66, which can be configured to monitor the volume of payload 64 in payload reservoir 62 and relay that information to microcontroller 31. In this embodiment, mouthpiece assembly 88 can be a consumable element that can be replaced as a complete subassembly once depleted, or simply interchanged with another mouthpiece assembly 88 containing a different payload 64 for consumption, depending on the needs and wants of the user. In some embodiments, oil gauge 66 can simply be a sight glass disposed on mouthpiece assembly 88 to provide a visual indicator to the user as to the amount of payload remaining therein. Atomizer assembly 79 is preferably configured to prevent air-lock and/or clogging with thick, undiluted payloads.

Control assembly 14 of vape device 100 is preferably substantially similar to control assembly 14 of vape device 10. Atomizer 20 of vape device 100 is preferably substantially similar to atomizer 20 of vape device 10, and may include alternative means for vaporizing a payload other than a heating element as described above in connection with vape device 10. It is within the scope of the invention for atomizer assembly 79 and mouthpiece assembly 88 to be formed integrally within a common housing that is releasably connected to control assembly 14. Further, it is within the scope of the invention for control assembly 14 and atomizer assembly 79 to be formed integrally within a common housing that is releasably connected to mouthpiece assembly 88. It is also within the scope of the invention for atomizer assembly 79, mouthpiece assembly 88, and control assembly 14 to be formed integrally within a common housing.

### Embodiments of Vape Device with Capacitive Vapor Measurement System

In some embodiments, the vape device includes a capacitive vapor measurement system to accurately gauge the concentration of a vaporized payload in a measurement cavity of the vape device so that the dosage of the vaporized payload can be accurately determined. The vapor measurement system includes a capacitive sensor (which may comprise a parallel plate capacitor, a rolled capacitor, a digitated capacitor, or other types of capacitors known in the art) in combination with a sensor measurement circuit configured to measure the capacitance of the capacitive sensor. The vapor measurement system also includes a processor programmed to determine the dosage of the vaporized payload based on the measured capacitance of the capacitive sensor. Various embodiments of the vapor measurement system are described below.

Referring to FIG. 4, one embodiment of a vape device 400 is shown that utilizes a capacitive sensor in the form of a parallel plate capacitor. Vape device 400 includes a housing 402 that defines an inlet 404 and an outlet 406 with an air flow chamber 408 that extends there between. An atomizer 410 is positioned in air flow chamber 408 and, as described above, atomizer 410 is in fluid communication with a payload reservoir (not shown). Atomizer 410 is configured to heat and vaporize the payload so as to output a vaporized payload 412.

A first conductive plate 414 and a second conductive plate 416 are positioned in air flow chamber 408 between atomizer 410 and outlet 406. Conductive plates 414 and 416 each comprise a generally square or rectangular plate that may be formed of metal or any other conductive material, such as copper, stainless steel, silicon (which may be doped), gold, or titanium. Conductive plates 414 and 416 are mounted or otherwise attached to the inner surface of housing 402 using a non-conductive mechanical support (not shown). Conductive plates 414 and 416 are spaced apart in a generally parallel relationship so as to define a measurement cavity 418 there between. As such, conductive plates 414 and 416 form a parallel plate capacitor that is used as the capacitive sensor of the vapor measurement system. When vaporized payload 412 passes from atomizer 410 to outlet 406, it passes through measurement cavity 418 such that vaporized payload 412 effectively functions as the dielectric of the parallel plate capacitor formed by conductive plates 414 and 416.

It should be understood that the components shown in FIG. 4 may be provided as part of an atomizer assembly of a vape device, may be provided as part of a replaceable cartridge of a vape device, or may be provided as part of an integrated vape device. Further, it should be understood that measurement cavity 418 may be placed at any position between the atomizer and the mouthpiece of a vape device.

The capacitance of the parallel plate capacitor formed by conductive plates 414 and 416 is expressed by the following equation:

$$C = \frac{\varepsilon_r \times \varepsilon_0 \times A}{d}$$

(1)

where

$C$ = capacitance of parallel plate capacitor in farads

$\varepsilon_r$ = relative dielectric constant of material between plates

$\varepsilon_0$ = permittivity of free space ($8.854 \times 10^{-12}$ farad/meter)

$A$ = area of plates in meters$^2$

$d$ = distance between plates in meters.

Because the relative dielectric constant of air is about 1 ($\varepsilon_r$ = 1.00059), any material with a dielectric constant greater than that of air that passes through measurement cavity 418 between conductive plates 414 and 416 will increase the capacitance of the parallel plate capacitor in a measurable manner. For example, the dielectric constant of vaporized payload 412 will typically be in the range of 2 to 10. This same principle applies to other types of capacitors that include first and second electrical conductors to form a capacitive sensor, as described below.

In one embodiment, the electrical conductors of the capacitive sensor (e.g., conductive plates 414 and 416) are coated with a protective film to maintain the integrity of the conductors (i.e., protect them from degradation due to a chemical reaction) and reduce the likelihood of a change in capacitance due to a buildup of condensate, which will change the dielectric constant of the material between the conductors.

In another embodiment, measurement cavity 418 is isolated within air flow chamber 408 by placing a guard on the side of measurement cavity 418 adjacent outlet 406. The guard is comprised of a conductor that is maintained at the same voltage as measurement cavity 418. When voltage is applied to conductive plates 414 and 416, a separate circuit applies the exact same voltage to the guard. Because there is no voltage difference between measurement cavity 418 and the guard, there is no electric field between them. Any conductors behind the guard will form an electric field with the guard instead of measurement cavity 418. Only the unguarded side of measurement cavity 418 adjacent atomizer 410 is allowed to form an electric field with vaporized payload 412. This guarding technique enables a more accurate measurement of the capacitance of the capacitive sensor.

There are many different electrical circuits that can be used to measure the capacitance of the capacitive sensor, both directly and indirectly, as known to those skilled in the art. In some embodiments, the sensor measurement circuit is configured to measure the absolute capacitance of the capacitive sensor when vaporized payload 412 passes through measurement cavity 418. In other embodiments, the sensor measurement circuit is configured to measure a capacitance shift, i.e., a change in capacitance of the capacitive sensor. In all of these embodiments, the measurement of the sensor's capacitance enables the dosage of the vapor's constituent parts to be accurately determined.

The vapor measurement system further includes a processor that is part of the control assembly or, alternatively, may be provided as part of the sensor measurement circuit. The processor is programmed to perform the following steps: (1) correlate the measured capacitance to a change in a dielectric constant; (2) correlate the change in the dielectric constant to a change in a dielectric density, i.e., the density of the vaporized payload; and (3) correlate the change in the dielectric density to a dosage of the vaporized payload. Thus, the measurement of the sensor's capacitance enables the dosage of the vapor's constituent parts to be accurately determined. The dose may be used to track and reduce consumption of a substance in the vaporized payload, as described below.

Because many types of payload are sticky, there will likely be a buildup of residue on the surfaces of conductive plates 414 and 416 (or other electrical conductors of a capacitive sensor, as described below) over time. In order to compensate for a capacitance shift due to this residue buildup, some embodiments utilize a calibration step in which the nominal capacitance of the capacitive sensor is measured (i.e., the capacitance when no vaporized payload is present in the measurement cavity) and used as a reference against which one or more subsequent capacitance measurements will be compared. When vaporized payload is passed through the measurement cavity, the capacitance of the capacitive sensor is remeasured. This second capacitance measurement is compared to the reference value and the difference between them is used to determine the concentration of vaporized payload in the measurement cavity. The calibration step may be performed on a periodic basis, e.g., before every capacitance measurement or every group of capacitance measurements. Of course, other calibration schedules will be apparent to one skilled in the art. For example, calibration steps may be performed both before and after the vaporized payload is present in the measurement cavity, in which case any new offset in the latter measurement is subtracted from the dose measured during inhalation insofar as the offset is attributable to new residue buildup on the sensor.

With reference again to vape device 400 shown in FIG. 4, the vapor measurement system of the present invention is not limited to the use of a capacitive sensor positioned in air flow chamber 408 between atomizer 410 and outlet 406. In some embodiments, the conductive plates of the capacitive sensor form a part of the atomizer, which enables the use of smaller vape devices that are more convenient to use. For example, one or both of the conductive plates of the capacitive sensor may function as a heating element/coil of the atomizer. The system can measure the pre-vape and post-vape capacitance of the capacitive sensor and the difference may be used to provide an accurate measurement of the dose administered.

As noted above, the vapor measurement system of the present invention is not limited to the use of a parallel plate capacitor as the capacitive sensor. Other types of capacitors may also be used, such as a rolled capacitor or an interdigitated capacitor. In general, any capacitor may be used that includes a first electrical conductor spaced from a second electrical conductor to define a measurement cavity there between.

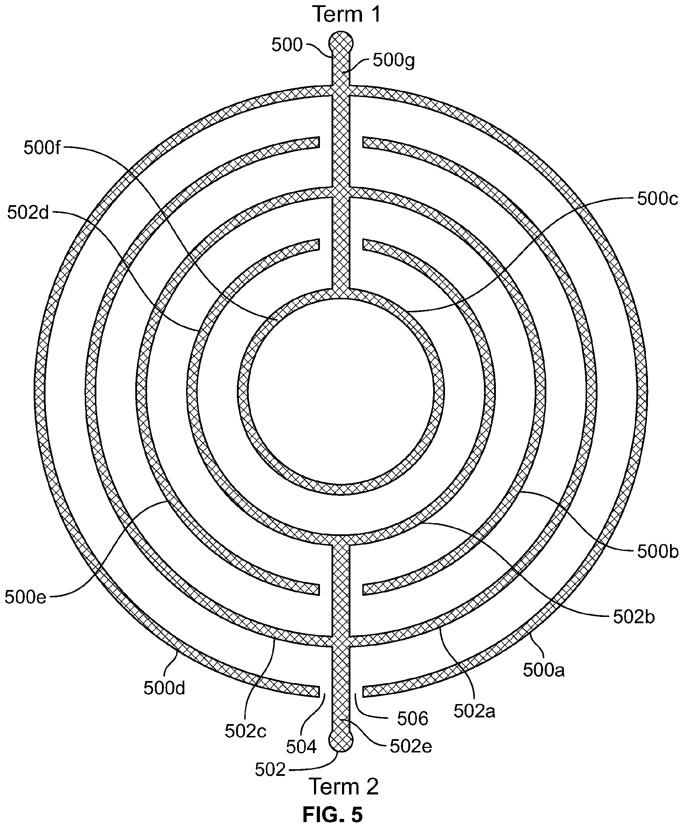

FIGS. 5-7 show an example of a rolled capacitor. As shown in FIG. 5, the rolled capacitor includes a first electrical conductor 500 and a second electrical conductor 502. First electrical conductor 500 comprises a plurality of rolled plates 500a-500f connected to a common mounting plate 500g that provides a first terminal connection (Term 1). Second electrical conductor 502 comprises a plurality of rolled plates 502a-502d connected to a common mounting plate 502e that provides a second terminal connection (Term 2). First and second electrical conductors 500 and 502 may be formed of metal or any other conductive material, such as copper, stainless steel, silicon (which may be doped), gold, or titanium.

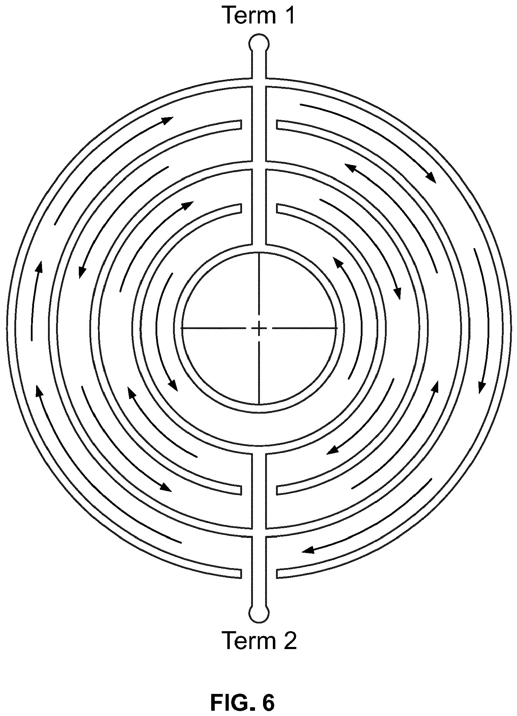

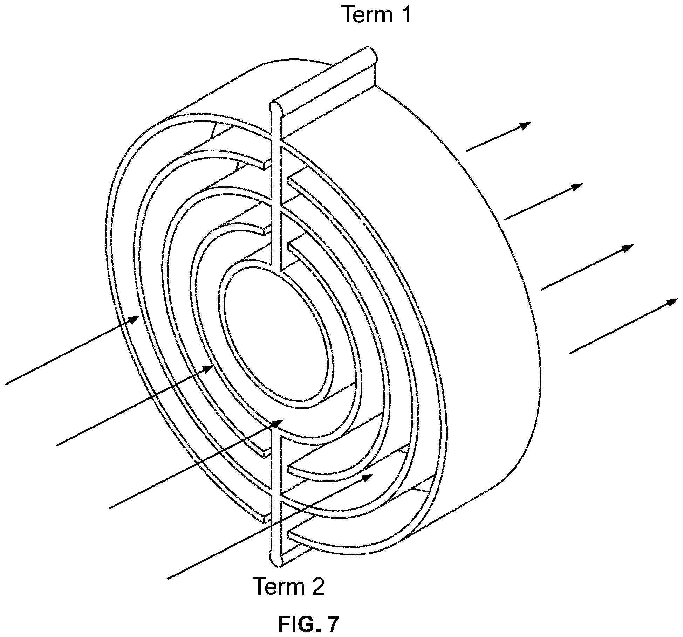

The rolled capacitor includes an air inlet 504 and an air outlet 506. FIG. 6 includes arrows indicating the direction of air flow in a plane parallel to the front face of the rolled capacitor. FIG. 7 includes arrows indicating the direction of air flow in a plane perpendicular to the front face of the rolled capacitor.

With reference to vape device 400 shown in FIG. 4, the rolled capacitor is configured to be positioned in air flow chamber 408 between atomizer 410 and outlet 406 (as an alternative to conductive plates 414 and 416) using a non-conductive mechanical support (not shown). First and second electrical conductors 500 and 502 define a measurement cavity between rolled plates 500a-500f and rolled plates 502a-502d. When vaporized payload 412 passes from atomizer 510 to outlet 506, it passes through the measurement cavity such that vaporized payload 412 effectively functions as the dielectric of the rolled capacitor.

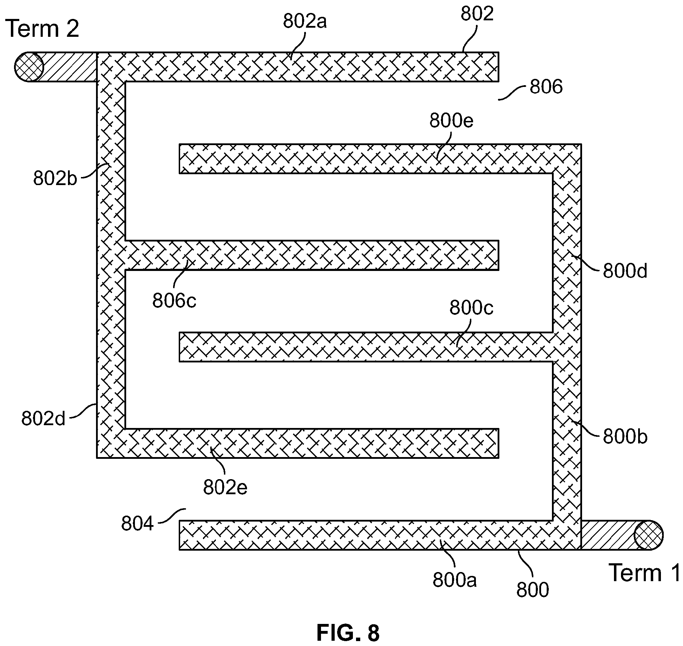

FIGS. 8-10 show an example of an interdigitated capacitor. As shown in FIG. 8, the interdigitated capacitor includes a first electrical conductor 800 and a second electrical conductor 802. First electrical conductor 800 comprises a plurality of interconnected segments 800a-800e that provide a first terminal connection (Term 1). Second electrical conductor 802 comprises a plurality of interconnected segments 802a-802e that provide a second terminal connection (Term 2). First and second electrical conductors 800 and 802 may be formed of metal or any other conductive material, such as copper, stainless steel, silicon (which may be doped), gold, or titanium.

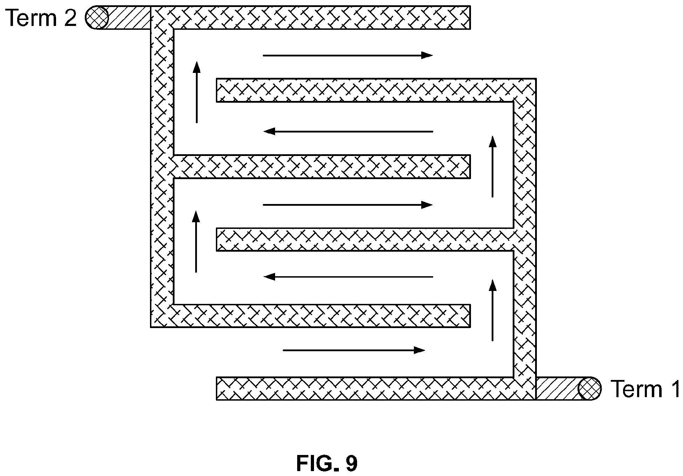

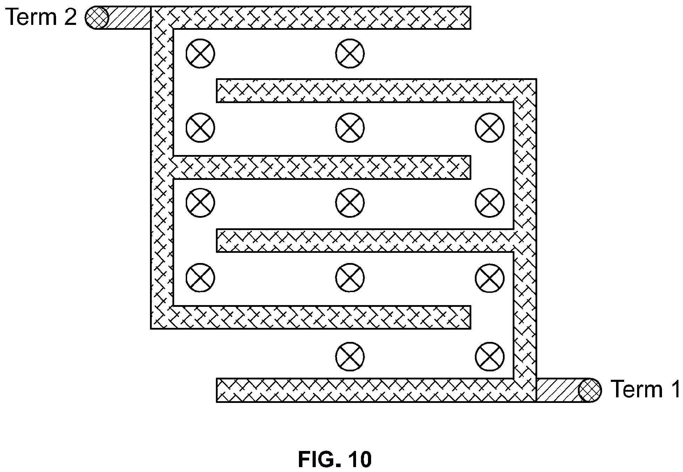

The interdigitated capacitor includes an air inlet 804 and an air outlet 806. FIG. 9 includes arrows indicating the direction of air flow in a plane parallel to the front face of the interdigitated capacitor. FIG. 10 includes arrows indicating the direction of air flow in a plane perpendicular to the front face of the interdigitated capacitor.

With reference to vape device 400 shown in FIG. 4, the interdigitated capacitor is configured to be positioned in air flow chamber 408 between atomizer 410 and outlet 406 (as an alternative to conductive plates 414 and 416) using a non-conductive mechanical support (not shown). First and second electrical conductors 800 and 802 define a measurement cavity between segments 800a-800e and segments 802a-802e. When vaporized payload 412 passes from atomizer 410 to outlet 406, it passes through the measurement cavity such that vaporized payload 412 effectively functions as the dielectric of the interdigitated capacitor.

It should be understood that multiple capacitors could be used in different configurations, including multiple parallel capacitors or multiple series capacitors. Of course, the use of multiple parallel capacitors is preferred because this configuration will increase the overall capacitance of the capacitive sensor and make it easier to detect (the overall capacitance would be reduced and harder to detect with multiple series capacitors).

In one embodiment, a modified differential interdigitated capacitive sensor design is used in which the electrical conductors of one capacitor are chemically modified and those of the other capacitor are not. The capacitors are positioned adjacent to each other so that substantially the same number of target molecules (e.g., nicotine, THC and/or CBD molecules) are present within each capacitor. The electrical conductors of the chemically modified capacitor have a coating designed to absorb the target molecules relative to the baseline capacitor. The surface absorption of the target molecules will alter the dielectric constant of the chemically modified capacitor, which may be measured by low noise electronics.

### Embodiments of Vape Device with Light Intensity Measurement System

In some embodiments, the vape device includes a light intensity measurement system to accurately determine the dosage of the vaporized payload. Various examples of vape devices that utilize different light intensity measurement systems will be described below. In each of these examples, the vape device includes at least one light sensor comprised of a light source and a light detector. The light source may comprise, for example, a light emitting diode (LED), a laser, or an incandescent lamp, although other types of light sources may also be used. The light detector may comprise, for example, a photo-diode, although other types of light detectors may also be used.

The characteristics of the light emitted by the light source for detection by the light detector will vary between different applications. The wavelength of the emitted light may fall in the visible or invisible (ultraviolet or infrared) portions of the electromagnetic spectrum. The emitted light may be continuous, or the light may be pulsed, for example, to save power or to take successive light intensity measurements. The pulsing is preferably scaled to reflect the air flow rate.

As discussed below, the light source and light detector are incorporated into a light intensity measurement circuit configured to obtain a plurality of light intensity measurements during each user inhalation (and optionally before and after user inhalation) and provide such measurements to the microcontroller of the vape device. Preferably, the characterization data used by the microcontroller to determine the vapor density based on the light intensity measurements will account for the vapor density changing with temperature and air flow rate.

In some examples, the light sensor is a reflective sensor in which the light source is configured to emit light that is directed toward the path of the vaporized payload and the light detector is configured to detect the light reflection from the vaporized payload (such as the light sensor incorporated into the vape device shown in FIG. 11). When the vapor density of the vaporized payload increases, the light reflection from the vaporized payload increases. Conversely, when the vapor density of the vaporized payload decreases, the light reflection from the vaporized payload decreases. These properties can be utilized to determine the vapor density of the vaporized payload.

With a reflective sensor, the wavelength of the emitted light may be selected so that the light is maximally reflected by the vaporized payload. The intensity of the emitted light is preferably low enough so that the reflected light does not overwhelm the light detector. In some embodiments, the light intensity is ramped over a very short time period (but slow enough that the photo-diode is able to track it) in order to identify the point at which the photo-diode begins to saturate. The photo-diode will saturate earlier if the vaporized payload has a higher vapor density and is more reflective. In some embodiments, the light intensity is dynamically modified based on the measured vapor density of the vaporized payload.

In other examples, the light sensor is a transmissive sensor in which the light source is configured to emit light that is directed toward the path of the vaporized payload and the light detector is configured to detect light transmission through the vaporized payload (such as the light sensors incorporated into the vape devices shown in FIGS. 12-15). When the vapor density of the vaporized payload increases, the light transmission through the vaporized payload decreases. Conversely, when the vapor density of the vaporized payload decreases, the light transmission through the vaporized payload increases. These properties can be utilized to determine the vapor density of the vaporized payload.

With a transmissive sensor, the wavelength of the emitted light may be selected so that the light is maximally absorbed by the vaporized payload. The intensity of the emitted light is preferably high enough to result in some light reaching the light detector. In some embodiments, the light intensity is ramped over a very short time period (but slow enough that the photo-diode is able to track it) in order to identify the point at which the photo-diode begins to saturate. The photo-diode will saturate earlier if the vaporized payload has a lower vapor density. In some embodiments, the light intensity is dynamically modified based on the measured vapor density of the vaporized payload.

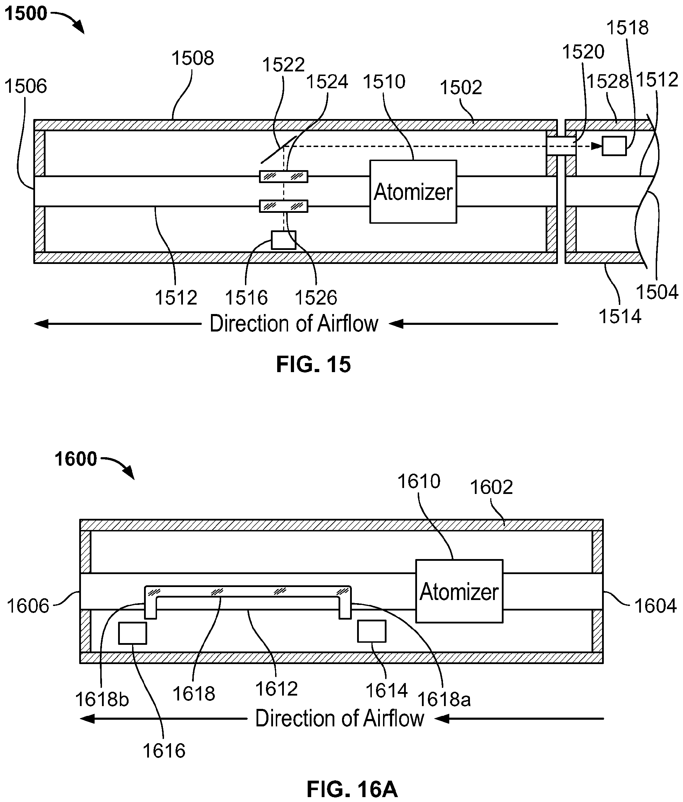

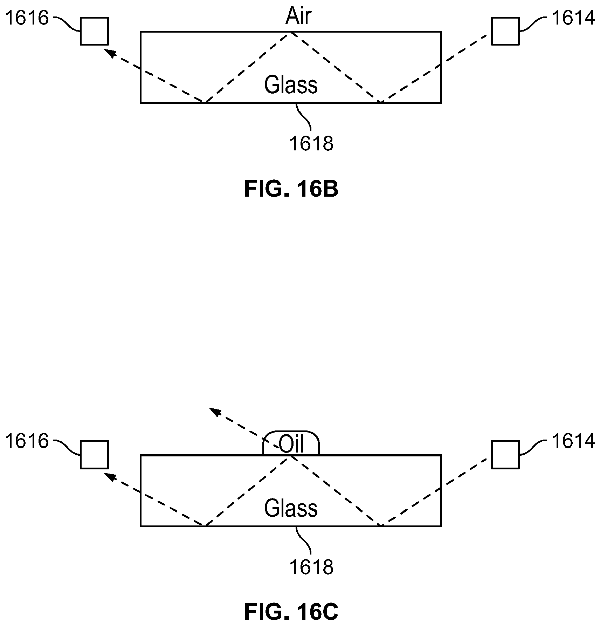

In yet other examples, the light source is configured to emit light that is directed toward a light transmitting medium positioned within the path of the vaporized payload and the light detector is configured to detect light transmitted through the medium (such as the light sensors incorporated into the vape devices shown in FIGS. 16-18). The light transmitting medium is made of glass, plastic or another material with an index of refraction that is sufficiently similar to that of the payload and sufficiently different from that of air.

When the surface of the light transmitting medium is surrounded entirely by air prior to any use of the vape device, most of the light emitted by the light source will travel through the medium to the light detector. Notably, when the light impacts the medium/air boundary at an angle, the light will totally reflect back into the medium (i.e., total internal reflection) due to the differences between the index of refraction of the medium and the index of refraction of air. Thus, the light detected by the light detector will have substantially the same intensity as the light emitted by the light source.

However, when vaporized payload, e.g., an oil droplet, is deposited on the surface of the light transmitting medium during use of the vape device, some of the light traveling through the medium will impact the medium/payload boundary at an angle and escape the medium into the deposited payload due to the similarities between the index of refraction of the medium and the index of refraction of the payload. Thus, the level of attenuation of the light received by the light detector will be dependent on the total amount of payload deposited on the surface of the medium.

It should be understood that the light transmission through the medium will decrease as the medium becomes increasingly fouled (coated) with droplets from the vaporized payload over the lifetime of the medium- i.e., there will be residual payload deposited on the surface of the medium after each user inhalation. Light intensity measurements are preferably obtained before, during, and after each user inhalation, and the rate of decrease of light transmission through the medium indicates the amount of vaporized payload that has passed the medium during that user inhalation. These properties can be utilized to determine the vapor density of the vaporized payload. Because the light transmitting medium has a limited lifetime, it is preferably placed in a replaceable cartridge portion of the vape device.

With this type of light sensor, the intensity of the emitted light is preferably increased over the lifetime of the light transmitting medium. When the medium is clean, very little of the emitted light will escape the medium due to total internal reflection at the medium/air boundary. As such, the emitted light can have a low intensity and still be detectable at the light detector. However, as droplets from the vaporized payload successively collect on the outside surface of the light transmitting medium throughout the lifetime of the medium, more light will escape the medium at the medium/payload boundary. As such, the emitted light must have a higher intensity to be detectable at the light detector.

The material of the light transmitting medium may be selected to obtain a desired index of refraction and associated effect. Those skilled in the art will appreciate that the critical angle is the smallest angle of incidence that yields total internal reflection through a light transmitting medium, as shown by the following equation:

$$\theta_c = \arcsin(n_2 / n_1)$$

$$(2)$$

where

$\theta_c$ = critical angle in degrees
$n_1$ = index of refraction of light transmitting medium
$n_2$ = index of refraction of material adjacent to light transmitting medium (e.g., the droplets from the vaporized payload).

The closer the indexes of refraction are between the light transmitting medium and the droplets from the vaporized payload, the more likely it will be that the light escapes the medium into one of the droplets. In some embodiments, the index of refraction of the light transmitting medium is selected so as to be substantially the same as the index of refraction of the vaporized payload so as to maximize the amount of escaped light. In other embodiments, the index of refraction of the light transmitting medium is selected so as to be slightly different than the index of refraction of the vaporized payload so as to limit the amount of escaped light, which may be beneficial in cases where it is desired to limit the amount of attenuation at the light detector. In other embodiments, the payload is modified via the use of additive(s) so as to obtain a desired index of refraction, although this approach is not preferred insofar as any such additive(s) may be inhaled by the user-i.e., it is preferred to modify the index of refraction of the light transmitting medium.

Of course, other vape devices may include any combination of the foregoing types of light sensors. For example, a vape device may include both a reflective sensor and a transmissive sensor that are used either simultaneously or sequentially to switch between the reflective and transmissive modes (depending on which mode provides better dynamic range).

Referring to FIG. 11, a first example of a vape device that relies on a light intensity measurement system to determine the dose of vaporized payload is shown generally as reference numeral 1100. Vape device 1100 includes a housing 1102, which may comprise an internal housing or external housing of vape device 1100. Positioned within housing 1102 is an air flow chamber which, in this example, comprises a conduit 1112 that extends between an inlet 1104 and an outlet 1106 that are capped to provide a sealed air path. It can be appreciated that the inlet and outlet orifices are defined by conduit 1112. An atomizer 1110 is positioned anywhere between inlet 1104 and outlet 1106. As described above, atomizer 1110 is configured to heat and vaporize the payload contained in a payload reservoir (not shown) so as to output a vaporized payload. During a user inhalation, ambient air flows through conduit 1112 from inlet 1104 to atomizer 1110, and ambient air mixed with vaporized payload flows through conduit 1112 from atomizer 1110 to outlet 1106. Outlet 1106 may further be in communication with a mouthpiece, as described above. Of course, it should be understood that vape device 1100 may include a number of other components that are not specifically shown in FIG. 11, including a power source, a microcontroller, and other electronics, as described above in connection with vape devices 10 and 100.

With respect to vape device 1100, the microcontroller is programmed to control the power source (e.g., a battery) so that the power source transmits a power signal (e.g., a direct current or pulsed direct current) to atomizer 1110 in accordance with desired operational settings. When the heating element of atomizer 1110 reaches the vaporization temperature of the payload contained in the payload reservoir, a portion of the payload is vaporized to thereby generate the vaporized payload for user inhalation. As described above, the microcontroller is programmed to determine the dose of vaporized payload based on a plurality of light intensity measurements obtained during user inhalation (and optionally before and after user inhalation), wherein the light intensity measurements are associated with light reflected from the vaporized payload when the vaporized payload passes through conduit 1112 from atomizer 1110 to outlet 1106.

As shown in FIG. 11, vape device 1100 includes a light source 1114 and a light detector 1116 positioned side-by-side within housing 1102 outside of conduit 1112 between atomizer 1110 and outlet 1106. In this example, conduit 1112 includes a transparent section 1118 formed on its sidewall that is located adjacent to light source 1114 and light detector 1116. Transparent section 1118 may be made of glass or any other transparent material known to those skilled in the art. As used herein, the term "transparent" generally means transparency for light and includes both clear transparency as well as translucency. Generally, a material is considered transparent if at least about 50%, preferably about 60%, more preferably about 70%, more preferably about 80% and still more preferably about 90% of the light illuminating the material can pass through the material.

The light path between light source 1114 and light detector 1116 is indicated by dashed lines in FIG. 11. As can be seen, light source 1114 is configured to emit light that passes through transparent section 1118 and into conduit 1112, whereby some of the light reflects off the vaporized payload within conduit 1112 and passes back through transparent section 1118 to light detector 1116 (noting that some of the emitted light will not be reflected back to light detector 1116). Light detector 1116 is then configured to generate a signal representing the intensity of the reflected light. As discussed above, light source 1114 and light detector 1116 are incorporated into a light intensity measurement circuit configured to obtain a plurality of light intensity measurements during each user inhalation (and optionally before and after user inhalation) and provide such measurements to the microcontroller of vape device 1100.

It should be understood that various modifications could be made to vape device 1100 within the scope of the present invention. For example, in some embodiments, light source 1114 and light detector 1116 are positioned within conduit 1112 (e.g., attached on a sidewall of conduit 1112) so that the vaporized payload can flow past light source 1114 and light detector 1116, provided that appropriate steps are taken to protect the integrity of the components within conduit 1112. In this case, transparent section 1118 of conduit 1112 would not be required. Of course, other modifications will be apparent to those skilled in the art.

Referring to FIG. 12, a second example of a vape device that relies on a light intensity measurement system to determine the dose of vaporized payload is shown generally as reference numeral 1200. Vape device 1200 includes a housing 1202, which may comprise an internal housing or external housing of vape device 1200. Positioned within housing 1202 is an air flow chamber which, in this example, comprises a conduit 1212 that extends between an inlet 1204 and an outlet 1206 that are capped to provide a sealed air path. It can be appreciated that the inlet and outlet orifices are defined by conduit 1212. An atomizer 1210 is positioned anywhere between inlet 1204 and outlet 1206. As described above, atomizer 1210 is configured to heat and vaporize the payload contained in a payload reservoir (not shown) so as to output a vaporized payload. During a user inhalation, ambient air flows through conduit 1212 from inlet 1204 to atomizer 1210, and ambient air mixed with vaporized payload flows through conduit 1212 from atomizer 1210 to outlet 1206. Outlet 1206 may further be in communication with a mouthpiece, as described above. Of course, it should be understood that vape device 1200 may include a number of other components that are not specifically shown in FIG. 12, including a power source, a microcontroller, and other electronics, as described above in connection with vape devices 10 and 100.

With respect to vape device 1200, the microcontroller is programmed to control the power source (e.g., a battery) so that the power source transmits a power signal (e.g., a direct current or pulsed direct current) to atomizer 1210 in accordance with desired operational settings. When the heating element of atomizer 1210 reaches the vaporization temperature of the payload contained in the payload reservoir, a portion of the payload is vaporized to thereby generate the vaporized payload for user inhalation. As described above, the microcontroller is programmed to determine the dose of vaporized payload based on a plurality of light intensity measurements obtained during user inhalation (and optionally before and after user inhalation), wherein the light intensity measurements are associated with light transmitted through the vaporized payload when the vaporized payload passes through conduit 1212 from atomizer 1210 to outlet 1206.

As shown in FIG. 12, vape device 1200 includes a light source 1214 and a light detector 1216 positioned within housing 1202 outside of conduit 1212 between atomizer 1210 and outlet 1206, wherein light source 1214 is positioned on a first side of conduit 1212 and light detector 1216 is positioned on a second opposing side of conduit 1212. In this example, conduit 1212 includes a first transparent section 1218 formed on its sidewall adjacent light source 1214 and a second transparent section 1220 formed on its sidewall adjacent light detector 1216. Transparent sections 1218 and 1220 may be made of glass or any other transparent material known to those skilled in the art.

The light path between light source 1214 and light detector 1216 is indicated by dashed lines in FIG. 12. As can be seen, light source 1214 is configured to emit light that passes through transparent section 1218 and into conduit 1212, whereby the light travels through the vaporized payload within conduit 1212 (noting that some of the light is absorbed by the vaporized payload) and passes through transparent section 1220 to light detector 1216. Light detector 1216 is then configured to generate a signal representing the intensity of the light that is received at light detector 1216. As discussed above, light source 1214 and light detector 1216 are incorporated into a light intensity measurement circuit configured to obtain a plurality of light intensity measurements during each user inhalation (and optionally before and after user inhalation) and provide such measurements to the microcontroller of vape device 1200.

It should be understood that various modifications could be made to vape device 1200 within the scope of the present invention. For example, in some embodiments, light source 1214 and light detector 1216 are positioned within conduit 1212 (e.g., attached on opposing sidewalls of conduit 1212) so that the vaporized payload can flow past light source 1214 and light detector 1216, provided that appropriate steps are taken to protect the integrity of the components within conduit 1212. In this case, transparent sections 1218 and 1220 of conduit 1212 would not be required. Of course, other modifications will be apparent to those skilled in the art.

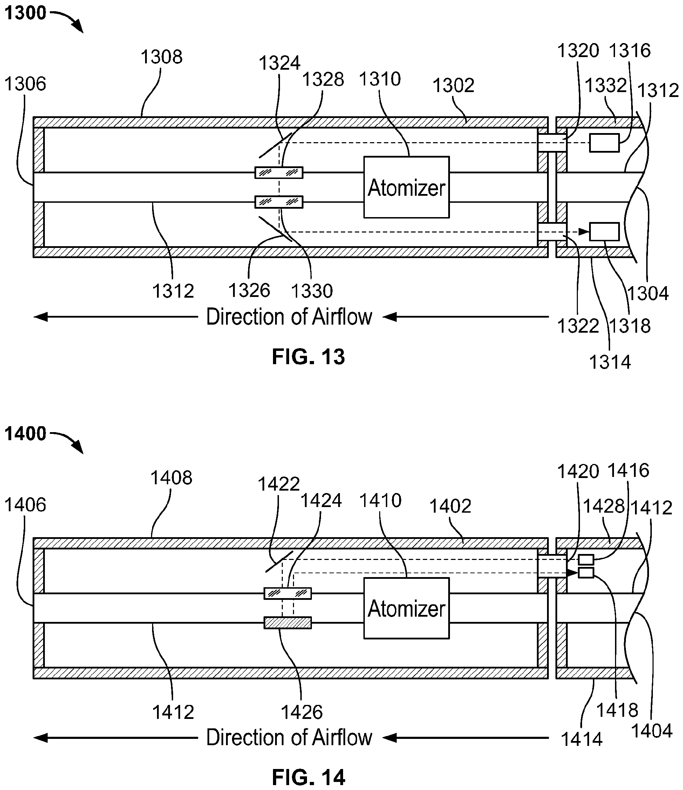

Referring to FIG. 13, a third example of a vape device that relies on a light intensity measurement system to determine the dose of vaporized payload is shown generally as reference numeral 1300. In this example, vape device 1300 includes a cartridge 1308 and a control assembly 1314 formed in separate housings 1302 and 1332, respectively, which are releasably connected to each other via an electromechanical connection, as described above. Housings 1302 and 1332 may comprise an internal housing or external housing of cartridge 1308 and control assembly 1314, respectively. Positioned within housings 1302 and 1332 is an air flow chamber which, in this example, comprises a conduit 1312 that extends between an inlet 1304 within control assembly 1314 and an outlet 1306 within cartridge 1308. Inlet 1304 and outlet 1306 are capped to provide a sealed air path (although the cap on inlet 1304 is not shown in FIG. 13). It can be appreciated that the inlet and outlet orifices are defined by conduit 1312. An atomizer 1310 is positioned anywhere between inlet 1304 and outlet 1306 within cartridge 1308. As described above, atomizer 1310 is configured to heat and vaporize the payload contained in a payload reservoir (not shown) so as to output a vaporized payload. During a user inhalation, ambient air flows through conduit 1312 from inlet 1304 to atomizer 1310, and ambient air mixed with vaporized payload flows through conduit 1312 from atomizer 1310 to outlet 1306. Outlet 1306 may further be in communication with a mouthpiece, as described above. Of course, it should be understood that vape device 1300 may include a number of other components that are not specifically shown in FIG. 13, including a power source, a microcontroller, and other electronics positioned in control assembly 1314, as described above in connection with vape devices 10 and 100. It should also be understood that conduit 1312 may be positioned entirely within cartridge 1308, in which case conduit 1312 would not extend through control assembly 1314 as shown.

With respect to vape device 1300, the microcontroller is programmed to control the power source (e.g., a battery) so that the power source transmits a power signal (e.g., a direct current or pulsed direct current) over the electromechanical connection to atomizer 1310 in accordance with desired operational settings. When the heating element of atomizer 1310 reaches the vaporization temperature of the payload contained in the payload reservoir, a portion of the payload is vaporized to thereby generate the vaporized payload for user inhalation. As described above, the microcontroller is programmed to determine the dose of vaporized payload based on a plurality of light intensity measurements obtained during user inhalation (and optionally before and after user inhalation), wherein the light intensity measurements are associated with light transmitted through the vaporized payload when the vaporized payload passes through conduit 1312 from atomizer 1310 to outlet 1306.

As shown in FIG. 13, vape device 1300 includes a light source 1316 and a light detector 1318 positioned within housing 1332 of control assembly 1314 outside of conduit 1312, wherein light source 1316 is positioned on a first side of conduit 1312 and light detector 1318 is positioned on a second opposing side of conduit 1312. Also, the interface between control assembly 1314 and cartridge 1308 includes a first transparent window 1320 located adjacent light source 1316 and a second transparent window 1322 located adjacent light detector 1318. In addition, a first reflective surface 1324 and a second reflective surface 1326 are located within housing 1302 of cartridge 1308. As can be seen, first reflective surface 1324 and first transparent window 1320 are positioned to align with light source 1316, and second reflective surface 1326 and second transparent window 1322 are positioned to align with light detector 1318. Further, conduit 1312 includes a first transparent section 1328 formed on its sidewall adjacent first reflective surface 1324 and a second transparent section 1330 formed on its sidewall adjacent second reflective surface 1326. Transparent sections 1328 and 1330 may be made of glass or any other transparent material known to those skilled in the art.

The light path between light source 1316 and light detector 1318 is indicated by dashed lines in FIG. 13. As can be seen, light source 1316 is configured to emit light that passes through first transparent window 1320 to first reflective surface 1324, whereby the light is reflected and redirected though first transparent section 1328 and into conduit 1312. The light then travels through the vaporized payload within conduit 1312 (noting that some of the light is absorbed by the vaporized payload) and passes through second transparent section 1330 to second reflective surface 1326, whereby the light is reflected and redirected through second transparent window 1322 to light detector 1318. Light detector 1318 is then configured to generate a signal representing the intensity of the light that is received at light detector 1318. As discussed above, light source 1316 and light detector 1318 are incorporated into a light intensity measurement circuit configured to obtain a plurality of light intensity measurements during each user inhalation (and optionally before and after user inhalation) and provide such measurements to the microcontroller of vape device 1300.

Referring to FIG. 14, a fourth example of a vape device that relies on a light intensity measurement system to determine the dose of vaporized payload is shown generally as reference numeral 1400. In this example, vape device 1400 includes a cartridge 1408 and a control assembly 1414 formed in separate housings 1402 and 1428, respectively, which are releasably connected to each other via an electromechanical connection, as described above. Housings 1402 and 1428 may comprise an internal housing or external housing of cartridge 1408 and control assembly 1414, respectively. Positioned within housings 1402 and 1428 is an air flow chamber which, in this example, comprises a conduit 1412 that extends between an inlet 1404 within control assembly 1414 and an outlet 1406 within cartridge 1408. Inlet 1404 and outlet 1406 are capped to provide a sealed air path (although the cap on inlet 1404 is not shown in FIG. 14).

It can be appreciated that the inlet and outlet orifices are defined by conduit 1412. An atomizer 1410 is positioned between inlet 1404 and outlet 1406 within cartridge 1408. As described above, atomizer 1410 is configured to heat and vaporize the payload contained in a payload reservoir (not shown) so as to output a vaporized payload. During a user inhalation, ambient air flows through conduit 1412 from inlet 1404 to atomizer 1410, and ambient air mixed with vaporized payload flows through conduit 1412 from atomizer 1410 to outlet 1406. Outlet 1406 may further be in communication with a mouthpiece, as described above. Of course, it should be understood that vape device 1400 may include a number of other components that are not specifically shown in FIG. 14, including a power source, a microcontroller, and other electronics positioned in control assembly 1414, as described above in connection with vape devices 10 and 100. It should also be understood that conduit 1412 may be positioned entirely within cartridge 1408, in which case conduit 1412 would not extend through control assembly 1414 as shown.

With respect to vape device 1400, the microcontroller is programmed to control the power source (e.g., a battery) so that the power source transmits a power signal (e.g., a direct current or pulsed direct current) to atomizer 1410 in accordance with desired operational settings. When the heating element of atomizer 1410 reaches the vaporization temperature of the payload contained in the payload reservoir, a portion of the payload is vaporized to thereby generate the vaporized payload for user inhalation. As described above, the microcontroller is programmed to determine the dose of vaporized payload based on a plurality of light intensity measurements obtained during user inhalation (and optionally before and after user inhalation), wherein the light intensity measurements are associated with light transmitted through the vaporized payload when the vaporized payload passes through conduit 1412 from atomizer 1410 to outlet 1406.

As shown in FIG. 14, vape device 1400 includes a light source 1416 and a light detector 1418 positioned in close proximity to each other within housing 1428 of control assembly 1414 outside of conduit 1412. Also, the interface between control assembly 1414 and cartridge 1408 includes a transparent window 1420 located adjacent light source 1416 and light detector 1418. In addition, a reflective surface 1422 is located within housing 1402 of cartridge 1408. As can be seen, reflective surface 1422 and transparent window 1420 are positioned to align with light source 1416 and light detector 1418. Further, conduit 1412 includes a transparent section 1424 formed on its sidewall adjacent reflective surface 1422 and a reflective section 1426 formed on an opposing sidewall. Transparent section 1424 may be made of glass or any other transparent material known to those skilled in the art. Reflective section 1426 may be made of polished stainless steel or any other reflective material known to those skilled in the art. If the section of conduit 1412 opposite transparent section 1424 is sufficiently reflective (e.g., if conduit 1412 is made of stainless steel), then a separate reflective section 1426 would not be required and that section of conduit 1412 would serve as the reflective section.

The light path between light source 1416 and light detector 1418 is indicated by dashed lines in FIG. 14. As can be seen, light source 1416 is configured to emit light that passes through transparent window 1420 to reflective surface 1422, whereby the light is reflected and redirected though transparent section 1424 and into conduit 1412. The light then travels through the vaporized payload to reflective section 1426, whereby the light is reflected and redirected back through the vaporized payload (noting that some of the light is absorbed by the vaporized payload). The light then passes through transparent section 1424 to reflective surface 1422, whereby the light is reflected and redirected through transparent window 1404 to light detector 1418. Light detector 1418 is then configured to generate a signal representing the intensity of the light that is received at light detector 1418. As discussed above, light source 1416 and light detector 1418 are incorporated into a light intensity measurement circuit configured to obtain a plurality of light intensity measurements during each user inhalation (and optionally before and after user inhalation) and provide such measurements to the microcontroller of vape device 1400.

Referring to FIG. 15, a fifth example of a vape device that relies on a light intensity measurement system to determine the dose of vaporized payload is shown generally as reference numeral 1500. In this example, vape device 1500 includes a cartridge 1508 and a control assembly 1514 formed in separate housings 1502 and 1528, respectively, which are releasably connected to each other via an electromechanical connection, as described above. Housings 1502 and 1528 may comprise an internal housing or external housing of cartridge 1508 and control assembly 1514, respectively. Positioned within housings 1502 and 1528 is an air flow chamber which, in this example, comprises a conduit 1512 that extends between an inlet 1504 within control assembly 1514 and an outlet 1506 within cartridge 1508. Inlet 1504 and outlet 1506 are capped to provide a sealed air path (although the cap on inlet 1504 is not shown in FIG. 15). It can be appreciated that the inlet and outlet orifices are defined by conduit 1512. An atomizer 1510 is positioned anywhere between inlet 1504 and outlet 1506 within cartridge 1508. As described above, atomizer 1510 is configured to heat and vaporize the payload contained in a payload reservoir (not shown) so as to output a vaporized payload. During a user inhalation, ambient air flows through conduit 1512 from inlet 1504 to atomizer 1510, and ambient air mixed with vaporized payload flows through conduit 1512 from atomizer 1510 to outlet 1506. Outlet 1506 may further be in communication with a mouthpiece, as described above. Of course, it should be understood that vape device 1500 may include a number of other components that are not specifically shown in FIG. 15, including a power source, a microcontroller, and other electronics positioned in control assembly 1514, as described above in connection with vape devices 10 and 100. It should also be understood that conduit 1512 may be positioned entirely within cartridge 1508, in which case conduit 1512 would not extend through control assembly 1514 as shown.

With respect to vape device 1500, the microcontroller is programmed to control the power source (e.g., a battery) so that the power source transmits a power signal (e.g., a direct current or pulsed direct current) to atomizer 1510 in accordance with desired operational settings. When the heating element of atomizer 1510 reaches the vaporization temperature of the payload contained in the payload reservoir, a portion of the payload is vaporized to thereby generate the vaporized payload for user inhalation. As described above, the microcontroller is programmed to determine the dose of vaporized payload based on a plurality of light intensity measurements obtained during user inhalation (and optionally before and after user inhalation), wherein the light intensity measurements are associated with light transmitted through the vaporized payload when the vaporized payload passes through conduit 1512 from atomizer 1510 to outlet 1506.

As shown in FIG. 15, vape device 1500 includes a light source 1516 positioned within housing 1502 of cartridge 1508 outside of conduit 1512 and a light detector 1518 positioned within housing 1528 of control assembly 1514 outside of conduit 1512. Also, the interface between control assembly 1514 and cartridge 1508 includes a transparent window 1520 located adjacent light detector 1518. In addition, a reflective surface 1522 is located within housing 1502 of cartridge 1508 outside of conduit 1512. As can be seen, reflective surface 1522 and transparent window 1520 are positioned to align with light detector 1518. Further, conduit 1512 includes a first transparent section 1524 formed on its sidewall adjacent reflective surface 1522 and a second transparent section 1526 formed on an opposing sidewall. Transparent sections 1524 and 1526 may be made of glass or any other transparent material known to those skilled in the art.

The light path between light source 1516 and light detector 1518 is indicated by dashed lines in FIG. 15. As can be seen, light source 1516 is configured to emit light that passes through second transparent section 1526 and into conduit 1512. The light travels through the vaporized payload within conduit 1512 (noting that some of the light is absorbed by the vaporized payload) and passes through first transparent section 1524 to reflective surface 1522, whereby the light is reflected and redirected though transparent window 1520 to light detector 1518. Light detector 1518 is then configured to generate a signal representing the intensity of the light that is received at light detector 1518. As discussed above, light source 1516 and light detector 1518 are incorporated into a light intensity measurement circuit configured to obtain a plurality of light intensity measurements during each user inhalation (and optionally before and after user inhalation) and provide such measurements to the microcontroller of vape device 1500.

Referring to FIG. 16A, a sixth example of a vape device that relies on a light intensity measurement system to determine the dose of vaporized payload is shown generally as reference numeral 1600. Vape device 1600 includes a housing 1602, which may comprise an internal housing or external housing of vape device 1600. Positioned within housing 1602 is an air flow chamber which, in this example, comprises a conduit 1612 that extends between an inlet 1604 and an outlet 1606 that are capped to provide a sealed air path. It can be appreciated that the inlet and outlet orifices are defined by conduit 1612. An atomizer 1610 is positioned anywhere between inlet 1604 and outlet 1606. As described above, atomizer 1610 is configured to heat and vaporize the payload contained in a payload reservoir (not shown) so as to output a vaporized payload. During a user inhalation, ambient air flows through conduit 1612 from inlet 1604 to atomizer 1610, and ambient air mixed with vaporized payload flows through conduit 1612 from atomizer 1610 to outlet 1606. Outlet 1606 may further be in communication with a mouthpiece, as described above. Of course, it should be understood that vape device 1600 may include a number of other components that are not specifically shown in FIG. 16A, including a power source, a microcontroller, and other electronics, as described above in connection with vape devices 10 and 100.

With respect to vape device 1600, the microcontroller is programmed to control the power source (e.g., a battery) so that the power source transmits a power signal (e.g., a direct current or pulsed direct current) to atomizer 1610 in accordance with desired operational settings. When the heating element of atomizer 1610 reaches the vaporization temperature of the payload contained in the payload reservoir, a portion of the payload is vaporized to thereby generate the vaporized payload for user inhalation. As described above, the microcontroller is programmed to determine the dose of vaporized payload based on a plurality of light intensity measurements obtained before, during, and after user inhalation, wherein the light intensity measurements in this example are associated with light transmitted through a light transmitting medium positioned parallel to the path of the vaporized payload within conduit 1612, as described below.

As shown in FIG. 16A, vape device 1600 includes a light source 1614 and a light detector 1616 spaced apart from each other within housing 1602 outside of conduit 1612 between atomizer 1610 and outlet 1606. Also, vape device 1600 includes one or more fibers made of glass, plastic, or another material with an index of refraction that is sufficiently similar to that of the payload and sufficiently different from that of air, as discussed above, which will be referred to herein as a "glass fiber 1618" for ease of reference. In this example, glass fiber 1618 is positioned substantially inside of conduit 1618 and extends generally parallel to the direction of the airflow. One end 1618a of glass fiber 1618 extends through an opening in the sidewall of conduit 1612 so as to be positioned outside of conduit 1612 adjacent light source 1614. The other end 1618b of glass fiber 1618 penetrates through another opening in the sidewall of conduit 1612 so as to be positioned outside of conduit 1612 adjacent light detector 1616. Any suitable sealant may be used to seal the openings in conduit 1612 so as to prevent the leakage of vaporized payload there through.

The light path between light source 1614 and light detector 1616 through glass fiber 1618 can be understood with reference to the simplified diagrams shown in FIGS. 16B and 16C.

FIG. 16B shows the surface of glass fiber 1618 surrounded entirely by air, i.e., prior to any use of vape device 1600. The light emitted by light source 1614 travels through glass fiber 1618 to light detector 1616 in a light path indicated by the dashed lines in FIG. 16B. As can be seen, when the light impacts each glass/air boundary at an angle, the light totally reflects back into glass fiber 1618 (i.e., total internal reflection) due to the differences between their respective indexes of refraction. Thus, the light detected by light detector 1616 has substantially the same intensity as the light emitted by light source 1614.

FIG. 16C shows the surface of glass fiber 1618 with vaporized payload (an oil droplet in this example) deposited on a portion of the surface. The light emitted by light source 1614 travels through glass fiber 1618 to light detector 1616 in a light path indicated by the dashed lines in FIG. 16C. As can be seen, when the light impacts the glass/oil boundary at an angle, the light will escape glass fiber 1618 and enter the oil, and some of the light may further escape the oil into the air. However, when the light impacts the glass/air boundary at an angle (i.e., in areas where there are no oil droplets on the surface), the light reflects back into glass fiber 1618. Thus, the level of attenuation of the light received by light detector 1616 will be dependent on the amount of oil deposited on the surface of glass fiber 1618.

It can be appreciated that light detector 1616 is configured to generate a signal representing the intensity of the received light. As discussed above, light source 1614 and light detector 1616 are incorporated into a light intensity measurement circuit configured to obtain a plurality of light intensity measurements before, during, and after each user inhalation and provide such measurements to the microcontroller of vape device 1600.

It should be understood that various modifications could be made to vape device 1600 within the scope of the present invention. For example, in some embodiments, glass fiber 1618 is positioned entirely inside of conduit 1612. In this case, a first transparent section is formed on the sidewall of conduit 1612 adjacent light source 1614 to provide a light path between glass fiber 1618 and light source 1614 and, similarly, a second transparent section is formed on the sidewall of conduit 1612 adjacent light detector 1616 to provide a light path between glass fiber 1618 and light detector 1616. The transparent sections may be made of glass or any other transparent material known to those skilled in the art. In this case, conduit 1612 would not need openings for the ends of glass fiber 1618. In yet other embodiments, glass fiber 1618 is replaced with another material having an index of refraction similar to that of the vaporized payload, as discussed above. Of course, other modifications will be apparent to those skilled in the art.

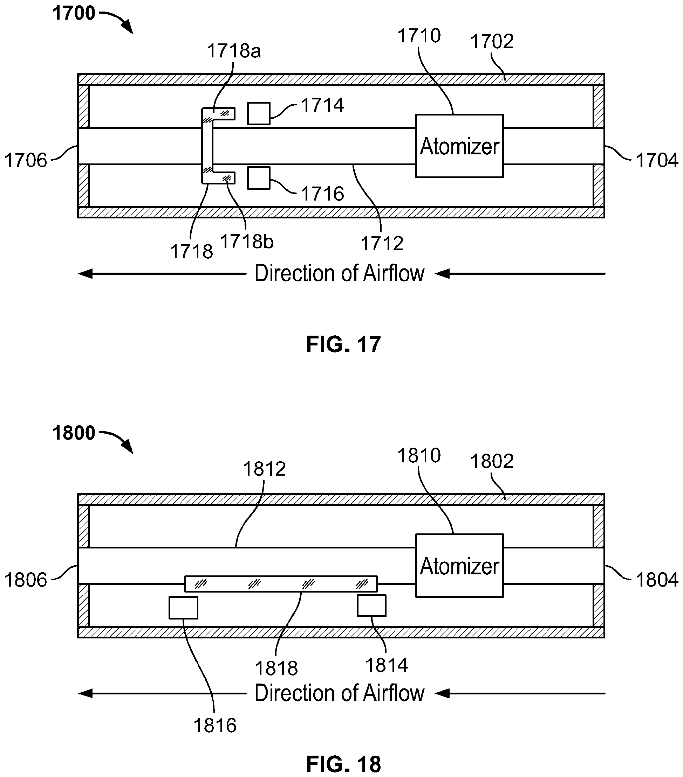

Referring to FIG. 17, a seventh example of a vape device that relies on a light intensity measurement system to determine the dose of vaporized payload is shown generally as reference numeral 1700. Vape device 1700 includes a housing 1702, which may comprise an internal housing or external housing of vape device 1700. Positioned within housing 1702 is an air flow chamber which, in this example, comprises a conduit 1712 that extends between an inlet 1704 and an outlet 1706 that are capped to provide a sealed air path. It can be appreciated that the inlet and outlet orifices are defined by conduit 1712. An atomizer 1710 is positioned anywhere between inlet 1704 and outlet 1706. As described above, atomizer 1710 is configured to heat and vaporize the payload contained in a payload reservoir (not shown) so as to output a vaporized payload. During a user inhalation, ambient air flows through conduit 1712 from inlet 1704 to atomizer 1710, and ambient air mixed with vaporized payload flows through conduit 1712 from atomizer 1710 to outlet 1706. Outlet 1706 may further be in communication with a mouthpiece, as described above. Of course, it should be understood that vape device 1700 may include a number of other components that are not specifically shown in FIG. 17, including a power source, a microcontroller, and other electronics, as described above in connection with vape devices 10 and 100.

With respect to vape device 1700, the microcontroller is programmed to control the power source (e.g., a battery) so that the power source transmits a power signal (e.g., a direct current or pulsed direct current) to atomizer 1710 in accordance with desired operational settings. When the heating element of atomizer 1710 reaches the vaporization temperature of the payload contained in the payload reservoir, a portion of the payload is vaporized to thereby generate the vaporized payload for user inhalation. As described above, the microcontroller is programmed to determine the dose of vaporized payload based on a plurality of light intensity measurements obtained before, during, and after user inhalation, wherein the light intensity measurements in this example are associated with light transmitted through a light transmitting medium positioned perpendicular to the path of the vaporized payload within conduit 1712, as described below.

As shown in FIG. 17, vape device 1700 includes a light source 1714 and a light detector 1716 positioned within housing 1702 outside of conduit 1712 between atomizer 1710 and outlet 1706, wherein light source 1714 is positioned on a first side of conduit 1712 and light detector 1716 is positioned on a second opposing side of conduit 1712. Also, vape device 1700 includes one or more fibers made of glass, plastic, or another material with an index of refraction that is sufficiently similar to that of the payload and sufficiently different from that of air, as discussed above, which will be referred to herein as a "glass fiber 1718" for ease of reference.. Glass fiber 1718 is positioned substantially inside of conduit 1712 and extends generally perpendicular to the direction of the airflow. One end 1718a of glass fiber 1718 extends through an opening in the sidewall of conduit 1712 so as to be positioned outside of conduit 1712 adjacent light source 1714. The other end 1718b of glass fiber 1718 penetrates through another opening in the sidewall of conduit 1712 so as to be positioned outside of conduit 12712 adjacent light detector 1716. Any suitable sealant may be used to seal the openings in conduit 1712 so as to prevent the leakage of vaporized payload there through.

The light path between light source 1714 and light detector 1716 through glass fiber 1718 can be understood from the description of FIGS. 16B and 16C above, wherein light detector 1716 is configured to generate a signal representing the intensity of the received light. As discussed above, light source 1714 and light detector 1716 are incorporated into a light intensity measurement circuit configured to obtain a plurality of light intensity measurements before, during, and after each user inhalation and provide such measurements to the microcontroller of vape device 1700.

It should be understood that various modifications could be made to vape device 1700 within the scope of the present invention. For example, in some embodiments, glass fiber 1718 is positioned entirely inside of conduit 1712. In this case, a first transparent section is formed on the sidewall of conduit 1712 adjacent light source 1714 to provide a light path between glass fiber 1718 and light source 1714 and, similarly, a second transparent section is formed on the sidewall of conduit 1712 adjacent light detector 1716 to provide a light path between glass fiber 1718 and light detector 1716. The transparent sections may be made of glass or any other transparent material known to those skilled in the art. In this case, conduit 1712 would not need openings for the ends of glass fiber 1718. In yet other embodiments, glass fiber 1718 is replaced with another material having an index of refraction similar to that of the vaporized payload, as discussed above. Of course, other modifications will be apparent to those skilled in the art.

Referring to FIG. 18, an eighth example of a vape device that relies on a light intensity measurement method to determine the dose of vaporized payload is shown generally as reference numeral 1800. Vape device 1800 includes a housing 1802, which may comprise an internal housing or external housing of vape device 1800. Positioned within housing 1802 is an air flow chamber which, in this example, comprises a conduit 1812 that extends between an inlet 1804 and an outlet 1806 that are capped to provide a sealed air path. It can be appreciated that the inlet and outlet orifices are defined by conduit 1812. An atomizer 1810 is positioned anywhere between inlet 1804 and outlet 1806. As described above, atomizer 1810 is configured to heat and vaporize the payload contained in a payload reservoir (not shown) so as to output a vaporized payload. During a user inhalation, ambient air flows through conduit 1812 from inlet 1804 to atomizer 1810, and ambient air mixed with vaporized payload flows through conduit 1812 from atomizer 1810 to outlet 1806. Outlet 1806 may further be in communication with a mouthpiece, as described above. Of course, it should be understood that vape device 1800 may include a number of other components that are not specifically shown in FIG. 18, including a power source, a microcontroller, and other electronics, as described above in connection with vape devices 10 and 100.

With respect to vape device 1800, the microcontroller is programmed to control the power source (e.g., a battery) so that the power source transmits a power signal (e.g., a direct current or pulsed direct current) to atomizer 1810 in accordance with desired operational settings. When the heating element of atomizer 1810 reaches the vaporization temperature of the payload contained in the payload reservoir, a portion of the payload is vaporized to thereby generate the vaporized payload for user inhalation. As described above, the microcontroller is programmed to determine the dose of vaporized payload based on a plurality of light intensity measurements obtained before, during and after user inhalation, wherein the light intensity measurements in this example are associated with light transmitted through a glass section of conduit 1812, as described below.

As shown in FIG. 18, vape device 1800 includes a light source 1814 and a light detector 1816 spaced apart from each other within housing 1802 outside of conduit 1812 between atomizer 1810 and outlet 1806. Also, in this example, conduit 1812 includes a flat or curved section made of glass, plastic, or another material with an index of refraction that is sufficiently similar to that of the payload and sufficiently different from that of air, as discussed above, which will be referred to herein as a "glass section 1818" for ease of reference. Glass section 1818 extends along the length of conduit 1812 such that one end of glass section 1818 is positioned adjacent light source 1814 and the other end of glass section 1818 is positioned adjacent light detector 1816.

The light path between light source 1814 and light detector 1816 through glass section 1818 can be understood from the description of FIGS. 16B and 16C above, wherein light detector 1816 is configured to generate a signal representing the intensity of the received light. As discussed above, light source 1814 and light detector 1816 are incorporated into a light intensity measurement circuit configured to obtain a plurality of light intensity measurements before, during and after each user inhalation and provide such measurements to the microcontroller of vape device 1800.

It should be understood that various modifications could be made to vape device 1800 within the scope of the present invention. For example, in some embodiments, a mirrored coating may be applied to the non-vapor side of glass section 1818, i.e., the outside surface of glass section 1818. In this case, light would still escape glass section 1818 when vaporized payload is deposited on the inside surface of glass section 1818. In other embodiments, all of conduit 1812 (not just glass section 1818) may be made of glass - either with or without a mirrored coating on the outside surface of conduit 1812. In yet other embodiments, glass section 1818 is made of another material having an index of refraction similar to that of the vaporized payload, as discussed above. Of course, other modifications will be apparent to those skilled in the art.

It should also be understood that any of vape devices 1600, 1700 and 1800 could be modified by placing a mirrored finish on the far end of the light transmitting medium from the light source so that the light sensor may be co-located with the light source. If the vape device includes a cartridge and a control assembly formed in separate housings that are releasably connected to each other via an electromechanical connection, as described above, this modification would enable the light source and/or the light detector to be positioned within the control assembly while the light transmitting medium is located within the cartridge (similar to the configurations shown in FIGS. 13-15).

Of course, other modifications to vape devices 1600, 1700 and 1800 will be apparent to those skilled in the art. For example, the glass fiber may be oriented at any angle within the conduit and is not limited to being positioned parallel to the direction of airflow (as in the vape device of FIG. 16A) or perpendicular to the direction of airflow (as in the vape device of FIG. 17). In addition, the glass fiber may follow the contour of the conduit, either in a direct line or helix.

Further, in each of the vape devices shown in FIGS. 11-18 above, the light intensity measurement circuit is configured to provide the light intensity measurements obtained during user inhalation (and optionally before and after user inhalation) to the microcontroller of the vape device. In some embodiments, the microcontroller is programmed to determine the dose of payload that is vaporized during each user inhalation by performing the following steps: (1) acquiring a plurality of light intensity measurements from the light intensity measurement circuit during user inhalation (and optionally before and after user inhalation) and (2) using the information from step 1 to determine the vapor density of the vaporized payload and by extension the partial mass of the payload that is vaporized during user inhalation.

In some embodiments, the method may be further refined (optionally) by varying the intensity of the light signal emitted by the light source and/or the gain of the light detector in order to adjust the sensitivity.

In some embodiments, the method may be further refined (optionally) by applying an electric field to the air flow chamber so as to orient a plurality of molecules in the vaporized payload when the vaporized payload passes through the conduit in order to improve their reflective properties.

Finally, it should be understood that all or a portion of the processing steps performed by the microcontroller of the vape device, as described above, could alternatively be performed by one or more other microcontrollers, such as a secondary microcontroller positioned in a cartridge of the vape device (for embodiments in which the vape device comprises a cartridge releasably connected to a control assembly). Various embodiments will be apparent to those skilled in the art.

### *General Operation of Vape Device*

The general operation of a vape device in accordance with some embodiments of the present invention will now be described with reference to the block diagrams shown in FIGS. 19 and 23. It should be understood that these methods may be implemented with any of the vape devices described above; provided, however, that minor modifications would need to be made in order to implement the method described in connection with FIG. 23 insofar as the vape device includes two separate atomizers/payload reservoirs and dose measuring devices for two different payloads, as described below. It should also be understood that other embodiments of the present invention will not include all of the functions and features described in connection with FIGS. 19 and 23.

FIG. 19 illustrates the general components of a vape device 1900 that may be used to vaporize a single payload that includes, for example, nicotine or THC. In operation, when fresh air is drawn into an air intake 1902, a pressure sensor 1906 measures a drop in pressure relative to the ambient pressure (if comparing using differential pressure across a membrane) or alternatively a sharp drop in pressure over a short period of time (if comparing in time using absolute air pressure) and acts as a trigger for a microcontroller 1914. Microcontroller 1914 causes an atomizer 1908 to heat and vaporize a payload 1912, and the vaporized payload passes into a dose measuring device 1910 prior to exiting the device via a vapor outlet 1904. In some embodiments, dose measuring device 1910 comprises one of the capacitive vapor measurement systems described above in connection with the vape devices shown in FIGS. 4-10. In other embodiments, dose measuring device 1910 comprises one of the light intensity measurement systems described above in connection with the vape devices shown in FIGS. 11-18. Of course, other types of systems configured to measure the dose of the vaporized payload may also be used. Microcontroller 1914 transmits the dose information to a computing device via a wireless transceiver 1916. This method is then repeated for each of the user inhalations.

As described below, the computing device will receive the dose information associated with each of the user inhalations during a calibration period and will also enable the user to set a goal to reduce consumption of payload 1912. The computing device then analyzes the dose information and desired goal to determine a dosage schedule to be followed during a substance reduction period, i.e., a schedule of metered doses designed to gradually reduce consumption of payload 1912 over a period of time in order to meet the goal. The computing device controls operation of the vape device in accordance with the dosage schedule so that the desired amount of payload 1912 is vaporized by atomizer 1908 for each user inhalation. In some embodiments, the computing device controls operation of the vape device by transmitting all or a portion of the dosage schedule to microcontroller 1914 via wireless transceiver 1916 (wherein microcontroller 1914 communicates with dose measuring device 1910 to ensure that the exact dose specified in the dosage schedule is delivered to the user at the appropriate time). In other embodiments, the computing device controls operation of the vape device by transmitting a plurality of operational settings (e.g., temperature, time, duration, etc.) to microcontroller 1914 via wireless transceiver 1916, wherein the operational settings are selected to result in vaporization of the payload in accordance with at least a portion of the dosage schedule (wherein microcontroller 1914 utilizes the specified operational settings at the appropriate time to control the activation of atomizer 1908).

FIG. 23 illustrates the general components of a vape device 2300 that may be used to vaporize a first payload that includes, for example, nicotine, and a second payload that includes, for example, CBD. CBD is a non-psychoactive component of cannabis that has known anti-inflammatory and anti-anxiety effects when vaporized or consumed orally. By providing CBD in vaporized form at the same time as nicotine, the CBD vapor may be able to counter some of the withdrawal symptoms encountered when reducing nicotine usage. Over time, the CBD vapor may replace the reduced volume of nicotine consumed and the user is able to continue vaping but with a much healthier alternative. The same approach may also be used when vaporizing THC instead of nicotine.

In operation, when fresh air is drawn into an air intake 2302, a pressure sensor 2304 measures a drop in pressure relative to the ambient pressure (if comparing using differential pressure across a membrane) or alternatively a sharp drop in pressure over a short period of time (if comparing in time using absolute air pressure) and acts as a trigger for a microcontroller 2322. Microcontroller 2322 causes a first atomizer 2318 to heat and vaporize a first payload 2320 (e.g., a payload that includes nicotine or THC), and the vaporized payload passes into a first dose measuring device 2316. Microcontroller 2322 also causes a second atomizer 2306 to heat and vaporize a second payload 2308 (e.g., a payload that includes CBD), and the vaporized payload passes into a second dose measuring device 2310. As discussed above, each of dose measuring devices 2316 and 2310 may comprise a capacitive vapor measurement system, a light intensity measurement system, or any other type of system configured to measure the dose of the vaporized payload. Separate dose measuring devices are used for the two different payloads insofar as the operating parameters and/or sensing technology may be different to properly measure the concentration of the vapors. The vapors are then combined in mixer section 2312 prior to exiting the device via a vapor outlet 2314. Microcontroller 2322 transmits the dose information to a computing device via a wireless transceiver 2324. This method is then repeated for each of the user inhalations.

As described below, the computing device will receive the dose information associated with each of the user inhalations during a calibration period and will also enable the user to set a goal to reduce consumption of first payload 2320. The computing device then analyzes the dose information and desired goal to determine a dosage schedule to be followed during a substance reduction period, i.e., a schedule of metered doses designed to gradually reduce consumption of first payload 2320 over a period of time in order to meet the goal. The computing device utilizes the dosage schedule to control operation of microcontroller 2322 via wireless transceiver 2324 so that the desired amount of first payload 2320 is vaporized by first atomizer 2318 and the desired amount of second payload 2308 is vaporized by second atomizer 2306 for each user inhalation. In some embodiments, the computing device controls operation of the vape device by transmitting all or a portion of the dosage schedule to microcontroller 2322 via wireless transceiver 2324 (wherein microcontroller 2322 communicates with dose measuring devices 2316 and 2310 to ensure that the exact dose specified in the dosage schedule for each of the first and second payloads is delivered to the user at the appropriate time). In other embodiments, the computing device controls operation of the vape device by transmitting a plurality of operational settings (e.g., temperature, time, duration, etc.) to microcontroller 2322 via wireless transceiver 2324, wherein the operational settings are selected to result in vaporization of the first and second payloads in accordance with at least a portion of the dosage schedule (wherein microcontroller 2322 utilizes the specified operational settings at the appropriate time to control the activation of each of first and second atomizers 2318 and 2306 and thereby change the concentration of the two vapors).

## II. Computing Device and Vape Device Application

The system of the present invention includes a computing device in communication with a vape device (including any of the vape devices described above) for tracking, modifying and metering the dosage of vaporized payload delivered to a user so as to reduce the user's consumption of a substance over a period of time. The computing device may comprise any one of smart phones (such as those running on the iOS® and Android® operating systems, and others as well known to those skilled in the art), personal computing tablets (such as those made by Apple® and Samsung®, and by others as well known to those skilled in the art), personal computers, laptop computers, personal digital assistants, smart watches, fitness tracking wristbands, wearable devices, smart glasses, and any other electronic computing device known to those skilled in the art.

FIG. 3 shows one embodiment of a system 300 that includes a vape device 302 in communication with a computing device 304, which is illustrated as comprising a smart phone. Computing device 304 includes at least one processor and at least one memory device, wherein a set of instructions is stored in the memory device and executable by the processor to perform the functions described below. In this embodiment, the set of instructions is provided as a software application 306 that may be downloaded from an online store (e.g., the Apple® App Store® or Google Play®) and installed on computing device 304.

Computing device 304 also includes a means for communication with vape device 302 and optionally with a global telecommunications or computing network 308. In some embodiments, computing device 304 wirelessly communicates with vape device 302 via a radio frequency (RF) communications link 310. RF communications link 310 may be provided, for example, in accordance with the Bluetooth™ communications protocol, Wi-Fi™ IEEE 802 communications protocol, Zigbee IEEE 802.15.4-based protocol, and any other RF, short-range, and long-range communications protocol as well known to those skilled in the art. Computing device 304 may also communicate with vape device 302 via a wired connection.

Software application 306 running on computing device 304 enables a user to set-up a customized user profile and provides a variety of different operating modes, including one or more of a general operation mode, a calibration mode, a goal-setting mode, and a substance reduction mode. Each of these modes will be described in greater detail below. Of course, it should be understood that the invention is not limited to these particular modes-i.e., some embodiments will not include one or more of the modes described below and other embodiments will include additional modes that are not described below.

In some embodiments, the computing device comprises a stand-alone device that provides all or a portion of the operating modes described herein. In the event that the user is required to obtain a new device, the original device preferably includes communication means (e.g., an RF communications link as described above or a wired connection) that enables transfer of the relevant data (including the current user data, dose information, calibration data, dosage schedule, etc.) from the original device to the new device. This will eliminate the need to re-calibrate the new device and otherwise repeat the steps that have already been performed on the original device.

In some of the embodiments, the computing device is incorporated into the vape device itself such that the vape device provides all or a portion of the operating modes described herein. While these embodiments may not provide a high level of user interaction as compared to those that use a separate computing device (e.g., a smart phone) in communication with the vape device, the vape device may provide simplified indicators (e.g., LEDs or small LCD displays) that may be used to prompt the user to use the device, to enable user input/feedback (e.g., "I feel like I need nicotine" or "I don't feel like I need nicotine"), and/or to provide information to the user (e.g., the recommended strength of the next cartridge).

### General Operation Mode

In the general operation mode, computing device 304 is programmed to present the graphical user interface 312 shown in FIG. 3. Graphical user interface 312 includes a user information window 314 for displaying information regarding the operation of vape device 3302 in addition to general information. This general information can include general news as well as information on available updates for vape device 302 or software application 304 from the manufacturer or supplier of the same.

In some embodiments, graphical user interface 312 also includes a locate button 316 that enables the user to determine the location of vape device 302 should the user misplace it. By pressing locate button 316, computing device 304 can send a signal wirelessly to vape device 302 to operate an audible signal from an audio speaker or buzzer or other like device disposed thereon to assist the user in finding vape device 302.

In some embodiments, graphical user interface 312 further includes a heat swipe slider 318 that enables the user to manually control the heat used to vaporize the payload in vape device 302, wherein the signal transmitted by computing device 304 to vape device 302 to control the heat can be included in the operational settings. In some embodiments, graphical user interface 312 can also include a lock indicator 320, an unlock indicator 322 and a swipe slider 324 that permits a user to enable or disable vape device 302 by swiping slider 324 right or left, respectively.

Other features that enable the general operation of vape device 302 will be apparent to those skilled in the art.

### Calibration Mode

Some embodiments of the present invention include a calibration mode in which computing device 304 is programmed to present a graphical user interface that instructs the user to consume payload at a rate that is normal for his or her current daily activities. Every time the user consumes any payload, vape device 302 transmits dose information to computing device 304 (or stores the dose information for subsequent transmission to computing device 304) so that software application 306 can track usage and learn the user's consumption habits and patterns. It will be seen that the dose information may also be transmitted to computing device 304 during the substance reduction period, as described below.

The payload consumed by the user will typically include a number of constituent parts, including the substance to be tracked (e.g., nicotine or THC) and other substances such as vegetable glycerin (VG) and propylene glycol (PG). The dose information transmitted from vape device 302 to computing device 304 may comprise the mass of the substance being tracked (e.g., the mass of the vaporized nicotine or THC) or information from which the mass of that substance may be derived, as described in the examples provided below.

In some embodiments, payload information (e.g., the relative percentages of nicotine and other substances within the payload) is electronically stored in a memory of vape device 302. In this case, the microcontroller of vape device 302 is programmed to determine the mass of the vaporized nicotine based on the total mass of the vaporized payload and the relative percentage of nicotine within the payload. Thus, the dose information transmitted from vape device 302 to computing device 304 comprises the actual mass of the vaporized nicotine.

In other embodiments, payload information (e.g., the relative percentages of nicotine and other substances within the payload) is encoded in a barcode attached to vape device 302, and computing device 304 is used to decode the payload information from the barcode. In this case, the dose information transmitted from vape device 302 to computing device 304 comprises the total mass of the vaporized payload, and computing device 304 is programmed to determine the mass of the vaporized nicotine based on the total mass of the vaporized payload and the relative percentage of nicotine within the payload.

In yet other embodiments, a payload identifier is stored electronically in a memory device of vape device 302 (e.g., RFID tag 28 included in vape device 10 shown in FIG. 1 or any other type of memory device, such as an EEPROM) and the payload identifier is transmitted to computing device 304. A database is provided that associates a plurality of payload identifiers with their respective payload information (i.e., the relative percentages of each of the substances within the payload). The database may be stored locally on computing device 304, or may be stored on a remote server that may be accessed via global telecommunications or computing network 308. Computing device 304 is programmed to access the database in order to identify the payload information (e.g., the relative percentages of nicotine and other substances within the payload) associated with the payload identifier received from vape device 302. In this case, the dose information transmitted from vape device 302 to computing device 304 comprises the total mass of the vaporized payload, and computing device 304 is programmed to determine the mass of the vaporized nicotine based on the total mass of the vaporized payload and the relative percentage of nicotine within the payload. It should be understood that the payload identifier (which in some embodiments may comprise a unique payload identifier) may also be used to authenticate a cartridge of the vape device or for other purposes known to those skilled in the art.

Computing device 304 is programmed to store the received dose information in association with a time stamp for each user inhalation. In some embodiments, vape device 302 is able to provide real time upload of dose information to computing device 304, in which case computing device 304 is programmed to generate the time stamp upon receipt of the dose information from vape device 302. In other embodiments, vape device 302 will securely store the dose information for each of the user inhalations and upload it to computing device 304 at a later time, such as when vape device 302 is within range of computing device 304. In this case, the time stamp is generated by vape device 302 and transmitted to computing device 304 along with the dose information for each user inhalation.

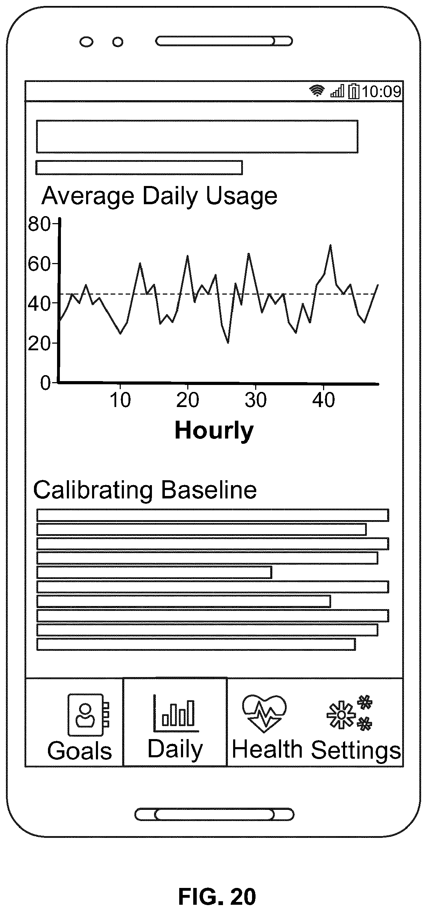

At the end of the calibration period (or even during the calibration period), computing device 304 is programmed to present the graphical user interface shown in FIG. 20, which includes the usage profile for the user. In this embodiment, the usage profile is shown in the form of a line graph in which the x-axis shows the time (in hours) and the y-axis shows the mass of the vaporized nicotine (in milligrams), wherein the peaks on the line graph correspond with the doses administered to the user. The user may also be able to reduce the time scale from hours to minutes, in which case the dose information may be presented as a bar chart to show the discrete consumption amounts. Of course, it should be understood that any type of graph may be used to show the amount of nicotine inhaled, the time of day at which the nicotine was inhaled, the time interval between user inhalations, and other parameters as will be apparent to those skilled in the art.

It should be understood that some embodiments of the present invention do not include a calibration mode in which dose information is transmitted from vape device 302 to computing device 304 during a calibration period. For example, in some embodiments, computing device 304 is programmed to present a graphical user interface that enables the user to provide historical usage information with details on the amount of nicotine that the user typically consumes (e.g., how many cigarettes in a time period; volume of a particular type of liquid and nicotine concentration in a time period, etc.). Computing device 304 may then analyze the historical usage information to determine the dosage schedule. As another example, in some embodiments, computing device 304 is programmed to present a graphical user interface that enables the user to select a desired dosage schedule (e.g., from a list of available dosage schedules). In this case, the dosage schedule may be modified during the substance reduction period (described below) if it is determined that the initial dosage schedule is not suitable for the user-e.g., if the user consistently "cheats" and does not follow the initial dosage schedule.

### Goal-Setting Mode

In the goal-setting mode, computing device 304 is programmed to present a graphical user interface that enables the user to set a goal for reducing consumption of a substance (e.g., nicotine or THC) to a specified level at the end of a specified period of time. In some embodiments, the specified level comprises a default level of 0 milligrams/day-i.e., complete cessation of all usage of the substance. In other embodiments, software application 306 enables the user to select the specified level, which may comprise any target amount (including amounts that are higher than 0 milligrams/day of the substance) that is reduced in comparison to the user's current average consumption.

In some embodiments, software application 306 enables the user to select the specified period of time to reach the specified level. In other embodiments, software application 306 is configured to recommend an appropriate period of time to reach the specified level based on information from smoking cessation research, historical data and/or other factors. For example, the recommended period of time will generally be longer for users with higher consumption rates compared to users with lower consumption rates. As another example, for a particular consumption rate, historical data may show that a one-month period of time yields a 20% success rate, a three-month period of time yields a 60% success rate, and a six-month period of time yields a 50% success rate. In this case, software application 306 may recommend a three-month period of time to reach the specified level. Of course, those skilled in the art will appreciate that other factors (e.g., current smoking/vaping rates, lifestyle, reason for quitting, etc.) may also be used to determine the recommended period of time.

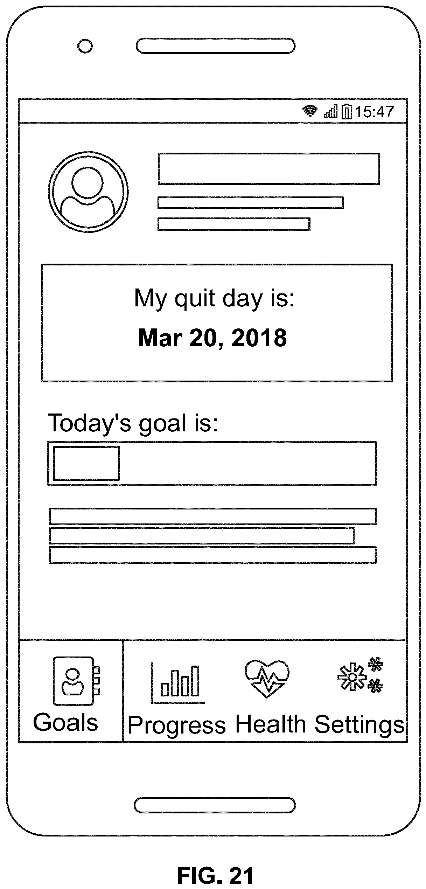

FIG. 21 illustrates an example of a graphical user interface presented by computing device 304 that shows the recommended target date to reach a specified level of a substance, such as nicotine (in this example, the specified level is the default of 0 milligrams/day). Of course, the user is preferably able to modify the recommended target date as desired. Also, the user is preferably able to return to this screen to track and modify the goals as desired during the duration of the process, as described below.

### Substance Reduction Mode

In the substance reduction mode, computing device 304 is programmed to analyze the dose information received during the calibration period (or the historical usage information provided by the user) to determine a dosage schedule that meets the goals set during the goal-setting period. The dosage schedule comprises a programmed sequence of specified doses of payload to be provided at specified times so as to reduce the amount of a substance (e.g., nicotine or THC) that is consumed by a user. For example, a dosage schedule that is designed to wean a user off nicotine would begin with doses that provide a relatively high nicotine content and gradually modify the doses to decrease the nicotine content over time. By reducing the nicotine consumption slowly, over time, withdrawal symptoms may be reduced and the user may be able to cease consumption of nicotine entirely.

In some embodiments, the dosage schedule is also designed to break existing habits by causing the user to consume payload at time intervals that are different and disruptive compared to his or her normal payload consumption and/or to provide the payload prior to normal yearning times in order to avoid over consumption or binging. For example, if the dose information received during the calibration period shows that a user typically consumes nicotine after dinner at 7:00 pm, the dosage schedule may break this habit by recommending consumption at 5:00 pm and then at 8:00 pm.

In some embodiments, the user is also dosed with bupropion, varenicline, naltrexone, or combinations thereof prior to, during, and/or after the substance reduction period.

In some embodiments, vape device 302 is configured to enable the vaporization of two different payloads, e.g., nicotine and CBD, and the dosage schedule is designed to begin with doses that provide a relatively high nicotine content and low CBD content and gradually modify the doses to increase the CBD content while decreasing the nicotine content. The dosage schedule modifies the respective concentrations of the nicotine and CBD over time so as to slowly replace the nicotine with CBD consumption. As such, the user can maintain existing consumption patterns by consuming CBD instead of nicotine. Once use of the nicotine has ceased, it is also possible to repeat the process to reduce any remaining habitual vaping that uses CBD as a payload substance.

In some embodiments, the two different substances, e.g., nicotine and CBD, are mixed together and provided in the same payload-e.g., within the same cartridge of the vape device. For these embodiments, the dosage schedule will dictate when to change cartridges within a series of one or more cartridges, wherein each cartridge includes pre-mixed concentrations of nicotine and CBD. For example, the first cartridge in the series may include a payload with a high concentration of nicotine and a low concentration of CBD (if any), and the last cartridge in the series may include a payload with a low concentration of nicotine (if any) and a high concentration of CBD. Preferably, vape device 302 or computing device 304 will alert the user when it is time to change the cartridge.

In some embodiments, computing device 304 is programmed to control operation of vape device 302 in accordance with the dosage schedule so that the desired amount of payload is vaporized during each user inhalation. At each specified time in the dosage schedule, vape device 302 or computing device 304 preferably provides an audio and/or visual alert to prompt the user to consume the specified dose of payload corresponding to the specified time.

In some embodiments, computing device 304 controls operation of vape device 302 by transmitting a vaporization signal to vape device 302 so as to cause the atomizer to vaporize the specified dose of the payload for the specified time. The vaporization signal may comprises the specified dose itself, or one or more operational settings that result in vaporization of the specified dose of the payload. It should be understood, however, that vape device 302 may be configured to operate without a continuous connection to computing device 304, as described below. This functionality may be provided as a setting in software application 306 to enable vape device 302 to enforce the dosage schedule without requiring access to computing device 304.

In other some embodiments, computing device 304 controls operation of vape device 302 by transmitting all or a portion of the dosage schedule to vape device 302. For example, computing device 304 may transmit a portion of the dosage schedule to vape device 302 daily, weekly, monthly or some other periodic basis in order to update vape device 302 with the most recent dosage schedule. Vape device 302 stores and implements the dosage schedule to ensure that the exact dose specified in the dosage schedule is delivered to the user at the appropriate time. For example, if the dosage schedule calls for delivery of x milligrams of nicotine at a certain time, vape device 302 will enable the atomizer and monitor the dose information received from a dose measuring device (e.g., any of the dose measurement systems described above), and then disable the atomizer when the specified dose has been reached.

In other embodiments, computing device 304 controls operation of vape device 302 by transmitting a plurality of operational settings (e.g., temperature, time, duration, etc.) to vape device 302, wherein the operational settings are selected to result in vaporization of the payload in accordance with at least a portion of the dosage schedule. For example, if the operational settings require that the atomizer is maintained at a specific temperature for a specific period of time (because it has been determined that those operational settings will result in the desired dose), vape device 302 will apply those operational settings at the appropriate time.

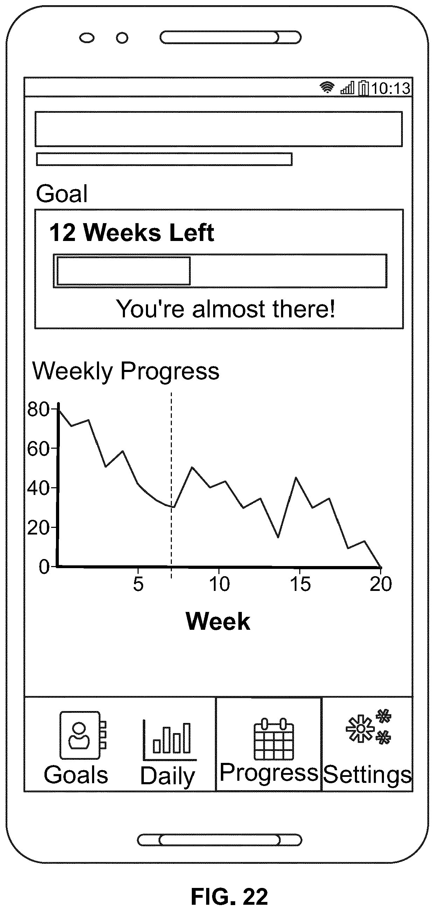

FIG. 22 illustrates an example of a graphical user interface presented by computing device 304 that shows the weekly progress of the user in relation to the dosage schedule. In this embodiment, the weekly progress is shown in the form of a line graph in which the x-axis shows the time (in weeks) and the y-axis shows the mass of the vaporized nicotine (in milligrams) consumed at a particular point in time. A vertical dashed reference line is used to reference the current time, wherein the data prior to the reference line comprises the user's actual consumption of nicotine and the data after the reference line comprises the recommended dosage schedule up until the end of the substance reduction period.

In some embodiments, the user is not permitted to use vape device 302 outside of the specified times in the dosage schedule. In other embodiments, the user is permitted to use vape device 302 outside of the specified times in the dosage schedule, in which case vape device 302 transmits dose information (described above) to computing device 304 during the substance reduction period. Computing device 304 is programmed to analyze this dose information and dynamically modify the dosage schedule to account for this use. The ability to use vape device 302 outside of the specified times in the dosage schedule may be provided as a setting in software application 306. Alternatively, computing device 304 may be programmed to present a graphical user interface that enables the user to modify the dosage schedule as desired.

### III. General Information

In this disclosure, the use of any and all examples or exemplary language (e.g., "for example" or "as an example") is intended merely to better describe the invention and does not pose a limitation on the scope of the invention. No language in the disclosure should be construed as indicating any non-claimed element essential to the practice of the invention.

Also, the use of the terms "comprises," "comprising," or any other variation thereof, are intended to cover a non-exclusive inclusion, such that a system, device, or method that comprises a list of elements does not include only those elements, but may include other elements not expressly listed or inherent to such system, device, or method.

Further, the use of relative relational terms, such as first and second, are used solely to distinguish one unit or action from another unit or action without necessarily requiring or implying any actual such relationship or order between such units or actions.

Finally, while the present invention has been described and illustrated hereinabove with reference to various exemplary embodiments, it should be understood that various modifications could be made to these embodiments without departing from the scope of the invention. Therefore, the present invention is not to be limited to the specific structural configurations, circuits or methodologies of the exemplary embodiments, except insofar as such limitations are included in the following claims.

**Claims**

1. A system for tracking and reducing consumption of a substance by a user, comprising:

   a vape device configured to enable consumption of a substance during a plurality of user inhalations, wherein the vape device comprises (a) a payload reservoir configured to contain a payload to be vaporized, wherein the payload includes the substance and (b) an atomizer configured to vaporize a portion of the payload during each of the user inhalations; and
   a software application executable on a computing device, wherein the software application causes the computing device to (a) determine a dosage schedule that causes a reduction in consumption of the substance over a period of time and (b) control operation of the vape device in accordance with the dosage schedule during a substance reduction period.

2. The system of claim 1, wherein the software application causes the computing device to receive dose information from the vape device, wherein the dose information comprises a mass of the substance vaporized by the atomizer during each respective user inhalation or information from which the mass of the substance may be derived.

3. The system of claim 2, wherein the information from which the mass of the substance may be derived comprises a mass of the payload vaporized by the atomizer during each respective user inhalation, and wherein the software application causes the computing device to (a) identify a relative percentage of the substance within the payload and (b) determine the mass of the substance based on the mass of the payload and the relative percentage of the substance within the payload.

4. The system of claim 3, wherein the software application causes the computing device to identify the relative percentage of the substance within the payload by (a) receiving payload information from the vape device, wherein the payload information includes the relative percentage of the substance within the payload, (b) decoding payload information from a barcode attached to the vape device, wherein the payload information includes the relative percentage of the substance within the payload, or (c) receiving a payload identifier from the vape device, wherein the payload identifier is associated in a database with payload information that includes the relative percentage of the substance within the payload.

5. The system of any of claims 1-4, wherein the software application causes the computing device to determine the period of time by (a) presenting a graphical user interface that enables the user to select the period of time or (b) analyzing research data to determine the period of time.

6. The system of any of claims 1-5, wherein the dosage schedule comprises a sequence of specified doses of the payload to be provided at specified times.

7. The system of claim 6, wherein the vape device or the computing device provides an alert to the user at each respective specified time.

8. The system of claims 6 or 7, wherein the software application causes the computing device to control operation of the vape device by (a) transmitting at least a portion of the dosage schedule to the vape device or (b) transmitting a plurality of operational settings to the vape device, wherein the operational settings result in vaporization of the payload in accordance with at least a portion of the dosage schedule.

9. The system of any of claims 2-8, wherein the software application causes the computing device to determine the dosage schedule by (a) analyzing the dose information received from the vape device during a calibration period to determine the dosage schedule or (b) presenting a graphical user interface that enables the user to provide historical usage information and analyzing the historical usage information to determine the dosage schedule.

10. The system of any of claims 2-8, wherein the software application causes the computing device to modify the dosage schedule by (a) presenting a graphical user interface that enables the user to modify the dosage schedule or (b) receiving the dose information from the vape device during the substance reduction period and modifying the dosage schedule based on the received dose information.

11. The system of any of claims 2-10, wherein the vape device is further configured to enable consumption of a second substance, wherein the dose information further comprises a mass of the second substance vaporized during each respective user inhalation or information from which the mass of the second substance may be derived, and wherein the dosage schedule causes an increase in consumption of the second substance over the period of time.

12. The system of any of claims 1-11, wherein the substance comprises nicotine or tetrahydrocannabinol (THC) and, optionally, the second substance comprises cannabidiol (CBD).

13. A computer-implemented method for tracking and reducing consumption of a substance by controlling an atomizer that enables vaporization of a payload that includes the substance during a plurality of user inhalations, the method comprising:

receiving dose information during a calibration period, wherein the dose information comprises a mass of the substance vaporized by the vape device during each respective user inhalation or information from which the mass of the substance may be derived;
analyzing the dose information received during the calibration period to determine a dosage schedule to be applied during a substance reduction period, wherein the dosage schedule comprises a sequence of specified doses of the payload to be provided at specified times so as to cause a reduction in consumption of the substance over a period of time; and
controlling operation of the atomizer in accordance with the dosage schedule during the substance reduction period.

14. The computer-implemented method of claim 13, further comprising the step of modifying the dosage schedule by (a) presenting a graphical user interface that enables the user to modify the dosage schedule or (b) receiving the dose information during the substance reduction period and modifying the dosage schedule based on the received dose information.

15. The computer-implemented method of claims 13 or 14, wherein the substance comprises nicotine or tetrahydrocannabinol (THC).

FIG. 1

**FIG. 2**

**FIG. 3**

**400**

418 414 410 402

406 408 412 410 408 404

416

Direction of Airflow

**FIG. 4**

Term 1

500

500g

500f

502d

500c

500e

500b

502b

500a

500d

502a

502c

506

504

502e

502

Term 2

**FIG. 5**

EP 3 644 320 A1

FIG. 6

31

Term 1

Term 2

**FIG. 7**

**FIG. 8**

**FIG. 9**

**FIG. 10**

**1100**

Atomizer

1110    1102

1106

1104

1118  1116   1114   1112

← Direction of Airflow ←

**FIG. 11**

**1200**

1220
1216          1210   1202

Atomizer

1206

1204

1218  1214      1212

← Direction of Airflow ←

**FIG. 12**

1300

1306 1308 1324 1328 1310 1302 1320 1316
1332 1312

Atomizer

1312 1326 1330 1304

Direction of Airflow ←

1322 1318

1314

**FIG. 13**

1400

1406 1408 1422 1424 1410 1402 1420 1416
1428 1412

Atomizer

1412 1426 1418 1404

Direction of Airflow ←

1414

**FIG. 14**

**FIG. 15**

**FIG. 16A**

FIG. 16B

FIG. 16C

**1700**

1718a

1710   1702

1714

Atomizer

1706

1704

1718  1718b   1712

1716

← — — — — Direction of Airflow ← —

**FIG. 17**

**1800**

1812

1810   1802

Atomizer

1806

1804

1818   1814

← — — — — Direction of Airflow ← —

1816

**FIG. 18**

**1900**

**FIG. 19**

Average Daily Usage

Hourly

Calibrating Baseline

Goals  Daily  Health Settings

**FIG. 20**

My quit day is:

**Mar 20, 2018**

Today's goal is:

Goals  Progress  Health  Settings

**FIG. 21**

**FIG. 22**

**2300**

2320   2318

2316

2302 →

2314 →

2304

2306   2308   2310   2312

2318

uController ←→ □

2322   2324

**FIG. 23**

EP 3 644 320 A1

Europäisches
Patentamt

European
Patent Office

Office européen
des brevets

# EUROPEAN SEARCH REPORT

**Application Number**

EP 19 20 5350

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2017/266397 A1 (MAYLE CHRIS G [US] ET AL) 21 September 2017 (2017-09-21) * paragraphs [0049], [0052], [0063]; figure 2 * | 1,11-13 | INV. G16H20/10 A24F40/00 |
| X | US 2018/093054 A1 (BOWEN ADAM [US] ET AL) 5 April 2018 (2018-04-05) * paragraphs [0071], [0133], [0166], [0179]; figure 1A * | 1,6-8,13 | |
| X | US 2016/089508 A1 (SMITH CALEB [US] ET AL) 31 March 2016 (2016-03-31) * paragraphs [0003], [0004], [0010], [0046], [0048], [0053], [0061], [0064], [0122]; figure 1 * | 1-5,9, 10,13-15 | |

TECHNICAL FIELDS
SEARCHED (IPC)

A24F
G16H

The present search report has been drawn up for all claims

| Place of search | Date of completion of the search | Examiner |
|---|---|---|
| Munich | 4 March 2020 | Schmidt, Karsten |

EPO FORM 1503 03.82 (P04C01)

**ANNEX TO THE EUROPEAN SEARCH REPORT
ON EUROPEAN PATENT APPLICATION NO.**                    EP 19 20 5350

04-03-2020

| Patent document cited in search report | | Publication date | Patent family member(s) | | Publication date |
|---|---|---|---|---|---|
| US 2017266397 | A1 | 21-09-2017 | CA | 3018731 A1 | 28-09-2017 |
| | | | EP | 3432740 A1 | 30-01-2019 |
| | | | US | 2017266397 A1 | 21-09-2017 |
| | | | WO | 2017165413 A1 | 28-09-2017 |
| US 2018093054 | A1 | 05-04-2018 | AU | 2017268810 A1 | 20-12-2018 |
| | | | BR | 112018074122 A2 | 06-03-2019 |
| | | | CA | 3025407 A1 | 30-11-2017 |
| | | | CN | 109963606 A | 02-07-2019 |
| | | | DE | 112017002267 T5 | 10-01-2019 |
| | | | EA | 201892738 A1 | 31-05-2019 |
| | | | EP | 3463535 A1 | 10-04-2019 |
| | | | GB | 2566185 A | 06-03-2019 |
| | | | JP | 2019521739 A | 08-08-2019 |
| | | | KR | 20190011264 A | 01-02-2019 |
| | | | PH | 12018502492 A1 | 15-07-2019 |
| | | | SG | 11201810438V A | 28-12-2018 |
| | | | TW | 201808373 A | 16-03-2018 |
| | | | US | 2018043114 A1 | 15-02-2018 |
| | | | US | 2018093054 A1 | 05-04-2018 |
| | | | WO | 2017205692 A1 | 30-11-2017 |
| US 2016089508 | A1 | 31-03-2016 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62751292